# FASCICULE DE BREVET EUROPEEN

(11) **EP 1 982 192 B1**
(45) Date de publication et mention de la délivrance du brevet: **26.03.2014**
(21) Numéro de dépôt: 07731536.4
(22) Date de dépôt: 30.01.2007
(51) Int. Cl.: A61K 39/395, A61K 31/713, A61K 38/17, G01N 33/574

(54) **PROCEDE DE DOSAGE DU ProNGF POUR LE DIAGNOSTIC IN VITRO DU CANCER EN PARTICULIER DU CANCER DU SEIN, DE LA THYROIDE, de la prostate, OU DU POUMON ET UTILISATION DU PRONGF EN THERAPIE**
VERFAHREN FÜR EIN PRO-NGF-ASSAY ZUR IN-VITRO-DIAGNOSE VON KREBS, INSBESONDERE BRUST-, SCHILDDRÜSEN-, Prostata- UND LUNGENKREBS, UND THERAPEUTISCHE ANWENDUNG VON PRO-NGF
METHOD FOR ProNGF ASSAY FOR IN VITRO DIAGNOSIS OF CANCER IN PARTICULAR BREAST , THYROID, prostate AND LUNG CANCER AND THERAPEUTIC USE OF ProNGF

(30) Priorité: 31.01.2006 FR 0600851
(43) Date de publication de la demande: 22.10.2008
(73) Titulaire: bioMérieux S.A., 69280 Marcy l'Etoile (FR); Université des Sciences et Technologies de Lille SAIC, 59655 Villeneuve d'Asq (FR)
(72) Inventeur: CHOQUET-KASTYLEVSKY, Geneviève, F-69340 Francheville (FR); DEMONT, Yohann, F-59800 Lille (FR); HONDERMARCK, Hubert, F-59650 Villeneuve d'Ascq (FR)
(74) Mandataire: Bitaud, Valérie Marie-Odile
(86) Numéro de dépôt international: PCT/FR2007/050708
(87) Numéro de publication internationale: WO 2007/088305

(56) Documents cités:
- WO-A-2004/040312
- WO-A-2004/056385
- WO-A-2005/076695
- WO-A1-2007/088305
- US-A1- 2001 046 959
- DESCAMPS S ET AL: "Nerve growth factor is mitogenic for cancerous but not normal human breast epithelial cells" JOURNAL OF BIOLOGICAL CHEMISTRY, AMERICAN SOCIETY OF BIOLOCHEMICAL BIOLOGISTS, BIRMINGHAM,, US, vol. 273, no. 27, 3 juillet 1998 (1998-07-03), pages 16659-16662, XP002277430 ISSN: 0021-9258
- TAGLIABUE E ET AL: "Nerve growth factor cooperates with p185HER2 in activating growth of human breast carcinoma cells" JOURNAL OF BIOLOGICAL CHEMISTRY, AMERICAN SOCIETY OF BIOLOCHEMICAL BIOLOGISTS, BIRMINGHAM,, US, vol. 275, no. 8, 25 février 2000 (2000-02-25), pages 5388-5394, XP002238474 ISSN: 0021-9258
- DOLLE L ET AL: "Nerve growth factor overexpression and autocrine loop in breast cancer cells" ONCOGENE, BASINGSTOKE, HANTS, GB, vol. 22, 28 août 2003 (2003-08-28), pages 5592-5601, XP002277431 ISSN: 0950-9232
- FAHNESTOCK MARGARET ET AL: "The precursor pro-nerve growth factor is the predominant form of nerve growth factor in brain and is increased in Alzheimer's disease" MOLECULAR AND CELLULAR NEUROSCIENCES, SAN DIEGO, US, vol. 18, no. 2, août 2001 (2001-08), pages 210-220, XP002294169 ISSN: 1044-7431

## Description

La présente invention concerne le domaine de la cancérologie. Plus particulièrement, la présente invention a pour objet un procédé de diagnostic du cancer et plus particulièrement du cancer du sein, de la thyroïde, du poumon ou de la prostate, chez un patient humain par détermination *in vitro* de la présence du précurseur du facteur de croissance nerveux (ProNGF) dans un échantillon biologique issu de ce patient, ou dans la tumeur du patient *in vivo,* ledit procédé pouvant être utilisé tant dans le diagnostic précoce, le dépistage, le suivi thérapeutique, le pronostic, que dans le diagnostic des rechutes dans le cadre du cancer. De plus, du fait de la capacité des cellules du cancer et plus particulièrement du cancer du sein, de la thyroïde, du poumon ou de la prostate à produire du ProNGF, la présente invention concerne également la thérapie.

Chez la femme, le cancer du sein est la première cause de mortalité par cancer dans les pays industrialisés. Il est estimé que la taille minimale d'une tumeur repérable par mammographie est de quelques millimètres (mm). Les cancers du sein se développent lentement. Néanmoins, cette tumeur de petite taille présente un passé évolutif de 8 ans en moyenne lors du diagnostic. L'étiologie du cancer du sein n'est pas bien définie. Des prédispositions familiales ont été mises en évidence. L'âge est le facteur de risque le plus important. Ainsi, le risque augmente de 0,5% par année d'âge dans les pays occidentaux. D'autres facteurs de risques sont connus tels que le nombre des grossesses et l'âge de la première grossesse, l'allaitement, l'âge de la puberté et de la ménopause, les traitements oestrogéniques après la survenue de la ménopause, le stress et la nutrition.

Le test disponible et utilisé en dépistage de masse pour le cancer du sein est une technique d'imagerie : la mammographie. Grâce à cette technique, la mortalité due aux cancers du sein a fortement diminué (30% de rédaction de mortalité), ce qui souligne l'importance du dépistage des tumeurs en terme de santé publique. Néanmoins, les techniques de dépistage souffrent d'un certain nombre de handicaps. La mammographie nécessite un matériel performant et du personnel qualifié ce qui est coûteux dans le cadre d'un dépistage de masse.

Le cancer de la thyroïde est un cancer rare. Il représente environ 1% des cancers survenant dans la population générale en France. Son incidence annuelle est faible soit environ 2,5 pour 100 000 personnes (Cancers : évaluation, traitement et surveillance. JM Andrieu & P Colonna Ed. ESTEM, Paris 1997). Le nombre de nouveaux cas de cancer de la thyroïde aux Etats-Unis en 2006 est estimé à 30.000, et les décès à 1500 (American Cancer Society.: Cancer Facts and Figures 2006. Atlanta, Ga: American Cancer Society, 2006).

Le cancer de la thyroïde se développe habituellement sous la forme d'un nodule situé au sein de la glande thyroïde qu'elle soit de taille normale ou augmentée (goitre). C'est un cancer rare, plus fréquent chez les sujets jeunes, et dont le pronostic est bon lorsque le cancer est dans sa forme papillaire puisque la guérison se produit dans 90 % des cas.

La prévalence du nodule thyroïdien est variable selon les moyens de dépistage. Il est plus fréquent chez la femme, le sujet âgé, les sujets vivant en zone de carence iodée ou ayant subi une irradiation de la région cervicale durant l'enfance mais ces nodules sont bénins dans plus de 90 % des cas. Les sujets jeunes sont plus exposés au développement du cancer du fait d'une plus grande sensibilité de la thyroïde à l'irradiation.

Suivant la classification histologique internationale, on distingue quatre types histologiques principaux de carcinome de la thyroïde :
- Les épithéliomas papillaires,
- les épithéliomas vésiculaires (ou folliculaires, terme anglo-saxon),
- les épithéliomas médullaires,
- les épithéliomas anaplasiques (ou indifférenciés).

Ces tumeurs peuvent être solitaires ou multifocales.
Les cancers papillaires sont les plus fréquents. Ils prédominent chez les sujets jeunes et représentent environ 80 % des cancers thyroïdiens.
Les cancers vésiculaires représentent environ 10 % des cancers thyroïdiens et sont surtout fréquents autour de la quarantaine.
Les cancers papillaires et vésiculaires représentent le groupe des cancers thyroïdiens différenciés radiosensibles. Ils sécrètent de la Thyroglobuline.
Les cancers médullaires représentent 5 % des cancers thyroïdiens et correspondent à une tumeur des cellules C ou parafolliculaires issues de la crête neurale. Les cellules C sécrètent la calcitonine.
Les cancers anaplasiques ou indifférenciés sont rares (moins de 5 % des cas) et d'une gravité extrême.
En présence d'un nodule thyroïdien malin, le traitement de base est la chirurgie:
S'il persiste du tissu fonctionnel résiduel, une dose d'iode 131 est administrée en chambre isolée, 4 à 6 semaines après une thyroïdectomie totale, afin de le stériliser en totalité.
Quarante pourcent environ des métastases des cancers thyroïdiens fixent l'iode et peuvent donc être traités par cette méthode.
Après thyroïdectomie totale et stérilisation de la thyroïde par l'iode 131, on administre de la thyroxine, hormone inhibitrice de la sécrétion de la TSH. Cette hormonothérapie permet également d'assurer un équilibre thyroïdien satisfaisant sur le plan fonctionnel. La découverte de nouveaux marqueurs diagnostiques, pronostique, et d'une thérapeutique ciblée, pourrait permettre de compléter l'arsenal thérapeutique et diagnostique de ce cancer.

Responsable de plus de 25 000 nouveaux cas chaque année en France, le cancer du poumon peut être considéré comme un problème majeur de santé publique. Cancer le plus fréquent chez l'homme, il représente en effet la première cause de mortalité par cancer chez les hommes et la troisième chez la femme. Le nombre de nouveaux cas de cancer de poumon (non-à petites cellules et à petites cellules combinés) aux Etats-Unis en 2006 est estimé à 174.470, et les décès à 162.460 (American Cancer Society.: Cancer Facts and Figures 2006. Atlanta, Ga: American Cancer Society, 2006).
A l'intérieur des cancers primitifs, l'examen des cellules cancéreuses (examen anatomopathologique) permet de distinguer :
- Les cancers épidermoïdes (35-40 %) ;
- Les adénocarcinomes (25-35 %) ;
- Les carcinomes à grandes cellules (10-15 %) ;
- Les carcinomes à petites cellules (20-25 %).
Ces quatre catégories représentent près de 95 % des cancers du poumon. Les trois premières sont regroupées en "carcinome non à petites cellules » (Non small cell lung cancer, NSCLC).
Le cancer à petites cellules évolue beaucoup plus rapidement et est plus susceptible de s'étendre à d'autres organes.
Plus rares, les tumeurs carcinoïdes et les tumeurs muco-épidermoïdes représentent les 1 à 2 % restants.
On peut résumer ces classifications en « cancer du poumon à petites cellules » (13%), ou « non à petites cellules » (87%), avec des implications thérapeutiques différentes. Les efforts de détection précoce n'ont pas fait la preuve de leur efficacité avec les outils actuels (Radio pulmonaire, analyse des crachats, fibroscopie n'améliorent pas la survie). Il est possible que le scanner spiralé ou les analyses moléculaires des crachats puisse permettre une détection plus précoce, avec des cancers plus facilement réséquables. Néanmoins aucun outil de dépistage n'a encore été découvert, du fait en particulier des risques rattachés aux biopsies pulmonaires et à la chirurgie, surtout chez les patients tabagiques.
Sans traitement, le carcer du poumon à petites cellules est la plus agressive des tumeurs pulmonaires, avec une survie médiane seulement de 2 à 4 mois. Comparé à d'autres types de cancer du poumon, le carcer du poumon à petites cellules a tendance à la dissémination avant le diagnostic mais il est plus sensible à la chimiothérapie et à la radiothérapie.
Le cancer poumon non-à petites cellules (NSCLC) recouvre différentes histologies. Les histologies les plus communes sont le carcinome épidermoïde où squameux, l'adénocarcinome, et le carcinome à grandes cellules. Ces histologies sont souvent classifiées ensemble parce que les approches au diagnostic, à la gradation, à l'établissement du pronostic, et au traitement sont semblables. Des patients présentant un cancer réséquable peuvent être guéris par chirurgie ou chirurgie avec chimiothérapie adjuvante. Le contrôle local de la maladie peut être réalisé avec la radiothérapie chez un grand nombre de patients avec un cancer non réséquable. Les patients présentant une maladie localement avancée et non réséquable peuvent avoir une survie à long terme avec radiothérapie combinée à la chimiothérapie. Les patients présentant la maladie métastatique avancée peuvent avoir une amélioration des symptômes et de la survie avec la chimiothérapie.
Au diagnostic, les patients avec NSCLC peuvent être divisés en 3 groupes de traitement similaire. Le premier groupe de patients comprend les tumeurs chirurgicalement réséquables (généralement stade I, stade II, et certains patients de stade III). Ce groupe a le meilleur pronostic. Les patients présentant un cancer réséquable avec des contre-indications médicales à la chirurgie sont des candidats pour la radiothérapie curative. Le deuxième groupe inclut des patients avec un cancer de poumon localement (T3-T4) et/ou régionalement (N2-N3) avancé.
Le troisième groupe inclut des patients avec les métastases à distance (M1) qui ont été trouvées au moment du diagnostic. Ce groupe peut être traité avec la radiothérapie ou la chimiothérapie palliative.
Des études multiples ont essayé d'identifier des facteurs pronostiques déterminants (Albain KS, Crowley JJ, LeBlanc M, et al.: Survival determinants in extensive-stage non-small-cell lung cancer: the Southwest Oncology Group expérience. J Clin Oncol 9 (9): 1618-26, 1991; Macchiarini P, Fontanini G, Hardin MJ, et al.: Blood vessel invasion by tumor cells predicts recurrence in completely resected T1 N0 M0 non-small-cell lung cancer. J Thorac Cardiovasc Surg 106 (1): 80-9, 1993; Ichinose Y, Yano T, Asoh H, et al.: Prognostic factors obtained by a pathologic examination in completely resected non-small-cell lung cancer. An analysis in each pathologic stage. J Thorac Cardiovasc Surg 110 (3): 601-5, 1995; Martini N, Bains MS, Burt ME, et al.: Incidence of local recurrence and second primary tumors in resected stage 1 lung cancer. J Thorac Cardiovasc Surg 109 (1): 120-9,1995; Fontanini G, Bigini D, Vignati S, et al.: Microvessel count predicts metastatic disease and survival in non-small cell lung cancer. J Pathol 177 (1): 57-63, 1995). Les facteurs qui sont corrélés avec un pronostic défavorable incluent ce qui suit :
- Présence de symptômes pulmonaires.
- Grande taille de la tumeur (>3 centimètres).
- Histologie non épidermoïde.
- Métastases ganglionnaires dans des ganglions établis par le TNM.
- Invasion vasculaire.

De même, des résultats contradictoires concernant l'importance pronostique de l'expression anormale d'un certain nombre de protéines dans des cancers de poumon ont été rapportés. Pour des patients présentant un cancer inopérable, le pronostic est compromis par un mauvais état général et une perte de poids de >10%.
Puisque le traitement n'est pas satisfaisant pour presque tous les patients avec NSCLC, il est nécessaire de découvrir de nouvelles cibles thérapeutiques, et de nouveaux outils de diagnostic précoce.

Le cancer de la prostate est le plus fréquent des cancers de l'homme de plus de 50 ans et représente la deuxième cause de décès par cancer chez l'homme dans le monde développé, après le cancer du poumon.

Son incidence augmente avec l'âge. En France, l'incidence globale en 1990 était de 71,4 pour 100 000 (2,6 dans la tranche 35-49 ans; 133,8 dans la tranche 50-69 ans; 726,9 dans la tranche des 70 ans). L'âge moyen du cancer de la prostate se situe autour de 70 ans mais certains hommes sont atteints à un âge plus précoce.

L'augmentation de 23 % de la mortalité liée au cancer prostatique, dans les vingt dernières années, reflète l'augmentation de l'espérance de vie et la reconnaissance plus fréquente du cancer de la prostate comme cause principale du décès. En France, la mortalité globale par cancer était de 33,4 pour 100 000 en 1990 soit plus de 9000 décès par an. Le cancer de la prostate représente 3,4% de tous les décès et 10,7% des décès par cancer.

Le cancer de la prostate se développe souvent très lentement, et reste localisé au début. Quand le cancer évolue, il peut s'étendre en dehors de la prostate par envahissement direct des tissus et des organes situés près de la prostate et il peut essaimer dans d'autres organes à distance de la prostate.

Le Prostate Spécifique Antigène (PSA) est un marqueur tumoral utilisé pour la détection du cancer de la prostate. Le taux de PSA dans le sang est exprimé en nanogrammes par millilitres (ng/ml) et est considéré comme normal si le taux est inférieur à 4 ng/ml. Plus le taux de PSA est élevé en cas de cancer de la prostate, plus le risque d'une extension à distance du cancer est élevé, ce qui signifie habituellement une diminution des chances de guérison ou de survie à long terme. Néanmoins, le PSA n'est pas le marqueur idéal : en effet certains cancers détectés par un taux de PSA augmenté auraient pu avoir une évolution très lente, sans avoir besoin de traitement. Il est donc indispensable de découvrir de nouveaux outils de diagnostic (au niveau tissulaire ou au niveau des fluides biologiques), afin de détecter précisément les cancers à potentiel agressif, à différencier des cancers d'évolution très lente.

Pour préciser le diagnostic, on utilise l'échographie endorectale de la prostate qui peut guider très précisément une aiguille pour faire des prélèvements à un endroit déterminé de la prostate. Seule la biopsie peut affirmer le cancer car les cellules cancéreuses sont visibles au microscope. Les biopsies ont donc une importance primordiale pour déterminer le pronostic de la maladie.

Le traitement de référence est la prostatectomie totale Cette opération enlève toute la prostate et les vésicules séminales. Elle n'est réalisée que si le cancer ne dépasse pas les limites de la prostate. Environ 10 % des patients vont développer une récidive locale dans les 5 ans suivant une prostatectomie radicale pour un cancer de la prostate localisé.

La radiothérapie est utilisée pour traiter les cancers qui sont localisés à la prostate, ou qui ont atteint les tissus voisins. Elle peut être utilisée pour diminuer le volume dé la tumeur ou éviter des complications locales.

Le but du traitement hormonal est de s'opposer à l'action des hormones mâles (androgènes) qui stimulent la prostate. Ainsi, la diminution du taux de testostérone, principale hormone masculine, bloque la prolifération des cellules cancéreuses et diminue le volume de la prostate. Le traitement hormonal n'a qu'un effet transitoire, il bloque la prolifération du cancer sans le guérir.

La chimiothérapie est utilisée dans le cancer de la prostate quand celui-ci a évolué avec une extension extraprostatique et qu'il ne répond plus au traitement hormonal. La chimiothérapie diminue la croissance tumorale et peut diminuer les douleurs liées au cancer.

Les traitements cités ci-dessus peuvent avoir un certain nombre d'effets secondaires, dont une incontinence urinaire, une impuissance, des troubles intestinaux (diarrhée, colite), et des troubles urinaires survenant essentiellement pendant le traitement (fréquence urinaire, diminution du jet, envies pressantes, brûlures en urinant, présence de sang dans les urines). Le traitement hormonal à long terme peut entraîner une ostéoporose avec fragilisation osseuse.

De nouvelles thérapeutiques ciblées pourraient permettre d'éviter un certain nombre de ces effets secondaires, et d'améliorer l'efficacité du traitement du cancer de la prostate. De nouveaux outils diagnostiques et pronostiques pourraient permettre de différencier les cancers d'évolution lente des cancers à potentiel métastatique, agressifs.

En pratique clinique, la caractérisation d'une tumeur en terme de malignité est réalisée après sa découverte par des méthodes histologiques dans des laboratoires spécialisés. Un ensemble de paramètres tels que la taille de la tumeur, son grade histopathologique, l'inflammation associée et l'invasion ganglionnaire sont utilisés pour décider de la thérapeutique et pour estimer le pronostic de la maladie.

Des marqueurs qui permettent de distinguer les cellules tumorales des cellules saines sont recherchés et étudiés depuis des années pour de nombreux cancers dont le cancer du sein, de la thyroïde, des poumons et de la prostate. Ils permettraient de diagnostiquer précocement la maladie, d'en établir le pronostic et la sensibilité au traitement, et d'en surveiller l'évolution. Jusqu'à présent les marqueurs candidats qui ont été identifiés et étudiés ont été des oncogènes, des marqueurs tissulaires et des marqueurs associés à l'angiogénèse ou aux capacités métastatiques de la tumeur. Actuellement les marqueurs de cancer du sein identifiés servent principalement pour le suivi thérapeutique. Il n'existe pas de test biologique validé pour le diagnostic précoce, ni pour le dépistage du cancer du sein, ni pour de nombreux autres cancers (pour le dépistage de masse du cancer colorectal, on dispose de la détection d'hémoglobine dans les selles). Le PSA peut être utilisé pour aider le diagnostic, et indiquer la nécessité d'une biopsie prostatique dans le cas du cancer de la prostate. Le dosage immunologique de la calcitonine dans le plasma est un excellent marqueur des cancers médullaires de la thyroïde. Dans certains pays, il peut être utilisé pour le dépistage du cancer de la prostate, mais il n'a pas été validé sur une base de population dans cette indication. Seule la détection des récepteurs aux oestrogènes sur le tissu tumoral mammaire permet de déterminer si les tumeurs du sein seront ou non hormono-sensibles. La détection du récepteur HER-2/neu dans les cancers du sein permet de savoir si la tumeur est sensible à l'Herceptine.

Un nombre limité de marqueurs antigéniques, en particulier le CA 15-3 (Basuyau, J. P., M. P. Blanc-Vincent, J. M. Bidart, A. Daver, L. Deneux, N. Eche, G. Gory-Delabaere, M. F. Pichon, and J. M. Riedinger. 2000. [Standards, Options and Recommendations (SOR) for tumor markers in breast cancer. SOR Working Group]. Bull Cancer. 87:723-37) a été identifié dans le cas des cellules mammaires cancéreuses. Ce marqueur est utilisé en pratique courante pour le suivi des patientes, en particulier pour la détection de récidive, mais, en raison de sa faible sensibilité, il n'est pas proposé en test de dépistage ni de diagnostic.

Depuis plusieurs années des travaux portant sur les antigènes associés au cancer du sein ont été développés non pas pour rechercher des marqueurs, mais pour rechercher des cibles pour une immunothérapie. Ceux-ci vont de la mise en évidence d'une immunité humorale contre les antigènes T/Tn (Springer, G. F. 1997. Immunoreactive T and Tn epitopes in cancer diagnosis, prognosis, and immunotherapy. J Mol Med. 75:594-602), jusqu'à la découverte plus récente d'anticorps et de réponses des cellules T dirigés contre p53 (Gnjatic, S., Z. Cai; M. Viguier, S. Chouaib, J. G. Guillet, and J. Choppin. 1998. Accumulation of the p53 protein allows recognition by human CTL of a wild-type p53 epitope presented by breast carcinomas and melanomas. J Immunol. 160:328-33) et HER-2/neu (Disis, M. L., and M. A. Cheever. 1997. HER-2/neu protein: a target for antigen-specific immunotherapy of human cancer. Adv Cancer Res. 71:343-71).

Plus récemment, une série de nouveaux antigènes potentiels a été mise en évidence par l'approche SEREX (serological expression cloning), reposant sur la construction de librairies d'ADNc de cellules tumorales et un screening par le sérum autologue. Un screening sérologique de librairie de cancer du sein a ainsi permis la mise en évidence de l'antigène *ING1* (Jager, D., E. Stockert, M. J. Scanlan, A. O. Gure, E. Jager, A. Knuth, L. J. Old, and Y. T. Chen. 1999. Cancer-testis antigens and ING1 tumor suppressor gene product are breast cancer antigens: characterization of tissue-specific ING1 transcripts and a homologue gene. Cancer Res. 59:6197-204.), puis d'un nouvel antigène de différenciation *NY-BR-1,* exprimé selon les auteurs dans 80% des cancers du sein et induisant la production d'anticorps IgG chez les patientes (Jager, D., E. Stockert, A. O. Gure, M. J. Scanlan, J. Karbach, E. Jager, A. Knuth, L. J. Old, and Y. T. Chen. 2001. Identification of a tissue-specific putative transcription factor in breast tissue by serological screening of a breast cancer library. Cancer Res. 61:2055-61). Ce type d'approche, qui a principalement servi à la recherche de cibles potentiellement utilisables pour le développement de vaccins, n'exclut pas *a priori* les antigènes présents dans le tissu normal (c'est le cas de *NY-BR-1*), ni ceux reconnus par un nombre limité de sérums de patientes (2/14 pour *ING1*), ils ne sont donc pas exploitables pour une stratégie de dépistage ou de diagnostic précoce. Par la même approche, d'autres antigènes induisant une réponse immunitaire humorale chez des patientes ont été signalés comme NY-BR-62, NY-BR-85 et la protéine D52. Ces antigènes seraient surexprimés respectivement dans 60%, 90% et 60% des cancers du sein (Scanlan, M. J., and D. Jager. 2001. Challenges to the development of antigen-specific breast cancer vaccines. Breast Cancer Res. 3:95-8.).

Les phénomènes moléculaires conduisant au développement des cancers, et plus particulièrement d'un cancer du sein impliquent des modifications de la structure et de l'expression d'oncogènes (tel que *ras*) et de mutations de gènes suppresseurs de tumeurs comme p53. La croissance des cellules tumorales dans la plupart des cancers du sein est dépendante des hormones oestrogéniques (oestradiol et progestérone) et de facteurs de croissance qui contrôlent la prolifération, la migration et l'apoptose. La croissance des cancers de la prostate est sous la dépendance des androgènes. La croissance de certains des cancers thyroïdiens est sous la dépendance de la Thyroide Stimulating Hormone (TSH). Ces facteurs de croissance soit stimulent, soit inhibent la prolifération, migration et différentiation des cellules tumorales de manière à agir de concert pour favoriser la croissance du cancer et les métastases. Par exemple, les facteurs de croissance de type insuline, le facteur de croissance de transformation as (TGF-α) et les facteurs de croissance du fibroblaste (FGF) peuvent tous stimuler la prolifération des cellules du cancer du sein, tandis que l'inhibiteur du facteur de croissance dérivé du sein (MDGI) et le facteur de croissance de transformation β (TGF-β) inhibent leur croissance.

Dans la demande de brevet WO2004/040312, les Demanderesses ont décrit l'utilisation du NGF en tant que marqueur tumoral et à titre de cible thérapeutique. Ainsi le NGF est produit par les cellules du cancer du sein, tandis que des cellules épithéliales mammaires normales correspondantes n'en produisent pas. De plus, le NGF stimule la survie et la prolifération des cellules épithéliales mammaires cancéreuses alors qu'il est sans effet sur les cellules épithéliales mammaires normales.

Le gène du NGF code un précurseur protéique appelé le ProNGF (26 kDa) qui, par clivage enzymatique, génère du NGF (13,6 kDa) (Seidah et al., 1996, Biochem J., 314 : 951-960). La principale source de NGF chez les mammifères est la glande sous maxillaire qui renferme uniquement du NGF et très peu de ProNGF. Pendant longtemps, le seul rôle accordé au ProNGF était un rôle de précurseur métabolique à durée de vie très transitoire.

Plus récemment, il a été montré dans différents tissus que le ProNGF était trouvé en quantité souvent supérieure à celle du NGF (Bierl et al., 2005, Neurosci Lett, 380 : 133-137). Hasan et al. ont décrit que le ProNGF peut être sécrété, ce qui signifie qu'il peut potentiellement agir de manière autocrine, paracrine ou même endocrine (2003, J Neurobiol, 57 : 38-53)..Enfin, récemment, il a été démontré que le ProNGF possède à la surface des cellules neuronales un site de fixation de haute affinité par lequel il a des effets propres, différents de ceux du NGF. Ainsi, le ProNGF est capable de se lier à la Sortiline, une glycoprotéine de 100 kDa (Mazella et al., 1998, J Biol Chem, 273 : 26273-26276), et d'induire l'apoptose de cellules neuronales *in vitro* (Nykjaer et al., 2004, Nature, 427: 843-848). Les demandes de brevet WO 2004/056385 et WO 2005/076695 montrent que l'interaction entre une neurotrophine (NGF ou ProNGF) peut être modulée à l'aide de composés particuliers, une telle modulation permettant le traitement des douleurs associées à certaines maladies du système génito-urinaire ou de patients dont le système nerveux a été endommagé. Il a aussi été attribué au ProNGF une activité pro-apoptotique *in vivo* (Pedraza et al., 2005, Am J Pathol, 166 : 533-543). Il est clair qu'il existe un découplage au niveau de l'expression et des effets biologiques du NGF et du ProNGF qui apparaissent être différents.

De façon plus générale, les propeptides ont longtemps été considérés comme étant uniquement des précurseurs métaboliques, un certain nombre d'exemple récents, notamment dans le domaine des neuropeptides, indiquent que dans certaines situations, les propeptides ont une activité biologique propre et dissociée de celle du peptide mature qu'ils peuvent générer. C'est clairement le cas du ProNGF qui peut être secrété par la cellule, possède ses récepteurs propres et est montré avoir des effets biologiques distinct de ceux du NGF sur les neurones. Le ProNGF constitue donc une entité moléculaire et biologique propre et différente du NGF. Certes, la séquence du NGF est contenue dans celle du ProNGF, mais leurs point isoélectriques et poids moléculaires sont différents ainsi que leurs activités biologiques.

Les Demanderesses ont maintenant mis en évidence de façon surprenante que les cellules épithéliales du cancer et en particulier du cancer du sein, de la thyroïde, du poumon et de la prostate produisent et sécrètent elles-mêmes du ProNGF, et ce en quantité notable, tandis que des cellules épithéliales saines des mêmes organes n'en produisent pas, de sorte que le ProNGF peut être utilisé à titre de marqueur tumoral ou bien à titre de cible thérapeutique. Les Demanderesses ont également établi que le ProNGF surexprimé dans les cellules cancéreuses possède une activité pro-métastatique pour ces cellules alors que le NGF a quant à lui une activité anti-apoptotique et mitogène.

Ainsi, la présente invention a pour premier objet un procédé de diagnostic *in vitro* du cancer, et en particulier des cancers du sein, de la thyroïde, du poumon ou de la prostate par détermination de la présence de ProNGF dans des échantillons biologiques issus de patients suspectés d'être atteints du cancer et en particulier des cancers du sein, de la thyroïde, du poumon ou de la prostate.

Le procédé de l'invention permet donc de diagnostiquer le cancer, et en particulier le cancer du sein, de la thyroïde, du poumon ou de la prostate par un test simple consistant à rechercher la présence de ProNGF dans un échantillon biologique prélevé d'un patient, ou dans la tumeur du patient *in vivo.* Les Demanderesses ont montré de façon inattendue que des cellules cancéreuses produisent du ProNGF, tandis que les cellules correspondantes non cancéreuses en sont incapables, comme cela sera mis en évidence de façon plus détaillée ci-après. Ainsi, la détermination de la présence de ProNGF dans l'échantillon permet de conclure à la pathologie recherchée, l'absence de ProNGF permettant de conclure à l'absence de pathologie. La présence de ProNGF au sein des tumeurs peut également être montrée *in vivo, in situ* dans les tumeurs.

Pour montrer la présence de ProNGF au sein d'une tumeur *in vivo,* on peut utiliser toute méthode d'imagerie connue de l'homme du métier. Pour cela, on peut coupler un partenaire de liaison du ProNGF à un traceur d'imagerie. Les partenaires de liaison spécifiques du ProNGF sont tout partenaire susceptible de se lier au ProNGF. A titre d'exemple, on peut citer les anticorps, les fractions d'anticorps, les récepteurs et toute autre molécule capable de se lier au ProNGF.

Les anticorps partenaires de liaison sont par exemple soit des anticorps polyclonaux, soit des anticorps monoclonaux.

Par couplage des partenaires de liaison à un traceur d'imagerie, on entend la fixation d'un traceur capable d'être détecté par toute méthode d'imagerie connue de l'homme du métier, et de générer directement ou indirectement un signal détectable. Ainsi, le traceur peut être un traceur radioactif comme le technétium-99. Dans ce cas, l'organe atteint du cancer primitif ou des métastases va fixer le ProNGF et son traceur. Le rayonnement émis par l'organe peut être filmé par une caméra spéciale, par exemple une gamma-caméra. L'appareil recueille les scintillations générées par la substance radioactive et permet ainsi de visualiser l'organe.
Dans un autre procédé de l'invention, le traceur comprendre un corps radioactif émettant des positrons (Fluor 18). Les images seront ensuite acquises par un système de Tomographie par Émission de Positrons.
Dans un autre procédé préféré de l'invention, le partenaire du ProNGF peut être couplé à des nanoparticules. A titre d'exemple, il peut s'agir de nanoparticules supramagnétiques. Par exemple des nanoparticules magnétiques anioniques pour l'application au marquage cellulaire direct et la détection *in vivo* par imagerie par résonance magnétique nucléaire. Il peut également s'agir de nanoparticules d'or. Grâce aux procédés de l'invention permettant la détection du ProNGF *in vivo,* on pourra visualiser les zones de l'organisme où il y a eu fixation du partenaire de liaison du ProNGF, les cancers produisant du ProNGF, et en particulier les cancers du sein, de la prostate, de la thyroïde et du poumon, ainsi que les localisations de leurs métastases à distance et les atteintes ganglionnaires.

La détermination de la présence de ProNGF *in vitro* peut être réalisée par détection directe du ProNGF, par culture de cellules sensibles au ProNGF, ou tout autre procédé de détermination de la présence d'une protéine dans un échantillon, connu de l'homme du métier.

La détermination de la présence de ProNGF par détection directe du ProNGF constitue un mode de réalisation particulier de l'invention.

Par détection directe du ProNGF, on entend la mise en évidence du ProNGF lui-même dans l'échantillon biologique.

La détection directe du ProNGF dans l'échantillon biologique peut être mise en oeuvre par tout moyen connu de l'homme du métier, comme par exemple par test immunologique ou par spectrométrie de masse, ce qui constitue un mode de réalisation particulier de l'invention.

Le test immunologique peut être tout test largement connu de l'homme du métier impliquant des réactions immunologiques, à savoir des réactions entre le ProNGF et un partenaire de liaison spécifique du ProNGF.

Les partenaires de liaison spécifiques du ProNGF sont tout partenaire susceptible de se lier au ProNGF. A titre d'exemple, on peut citer les anticorps, les fractions d'anticorps, les récepteurs et toute autre molécule capable de se lier au ProNGF.

Les anticorps partenaires de liaison sont par exemple soit des anticorps polyclonaux, soit des anticorps monoclonaux.

Les anticorps polyclonaux peuvent être obtenus par immunisation d'un animal avec du ProNGF, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment le ProNGF. Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec du ProNGF, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis du ProNGF pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Des exemples d'anticorps anti-ProNGF sont connus et sont disponibles notamment dans le catalogue de Chemicon.

Les partenaires de liaison spécifiques du ProNGF pourront être marqués pour la révélation de la liaison ProNGF/partenaire de liaison lorsque le partenaire de liaison est utilisé comme réactif de détection, et donc pour la détection directe du ProNGF dans l'échantillon biologique.

Par marquage des partenaires de liaison, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexas ou les phycocyanines.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand-qui porte le partenaire de liaison. L'anti-ligand peut être détectable directement par les marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de l'une des Demanderesses ou à l'article J. Histochem. Cytochem. 45 : 481-491, 1997.

Selon le type de marquage utilisé, l'homme du métier ajoutera des réactifs permettant la visualisation du marquage.

A titre d'exemple de tests immunologiques tels que définis ci-dessus, on peut citer les méthodes « sandwich » telles qu'ELISA, IRMA et RIA, les méthodes dites de compétition et les méthodes d'immunodétection directe comme l'immunohistochimie, l'immunocytochimie, le Westem-blot et le Dot-blot.

Dans le cas des méthodes dites de compétition, le ProNGF est marqué comme décrit précédemment pour le partenaire de liaison.

La spectrométrie de masse peut également être utilisée pour la détection directe du ProNGF dans l'échantillon biologique. Le principe de la spectrométrie est largement connu de l'homme du métier et est décrit par exemple dans Patterson, S., 2000, Mass spectrometry and proteomics. Physiological Genomics 2, 59-65.

Pour ce faire, l'échantillon biologique préalablement traité ou non est passé dans un spectromètre de masse et on compare le spectre obtenu avec celui du ProNGF. Un exemple de traitement préalable de l'échantillon consiste à le faire passer sur un support d'immunocapture, comportant un des partenaires de liaison du ProNGF, par exemple un anticorps dirigé contre le ProNGF. Un autre exemple de traitement préalable de l'échantillon peut être le pré-fractionnement de l'échantillon biologique, afin de séparer entre elles les protéines de l'échantillon. Dans des techniques bien connues de l'homme du métier, on peut par exemple tout d'abord dépléter les protéines, majoritaires de l'échantillon.

L'échantillon biologique utilisé pour la détection directe du ProNGF, susceptible de contenir du ProNGF en tant que tel, peut être constitué par du fluide biologique ou un tissu provenant de la biopsie de la tumeur ou des métastases du patient considéré.

A titre de fluide biologique, on peut citer le sang total ou ses dérivés tels que le sérum ou le plasma, la moelle osseuse, le lait, le liquide céphalo-rachidien, les urines et les épanchements. On préfère le sang ou ses dérivés.

Pour la détection du ProNGF, le fluide biologique, qui constitue un mode de réalisation particulier de l'invention, peut nécessiter un traitement particulier. En effet, le fluide biologique peut contenir le ProNGF en tant que tel, ou bien il peut contenir des cellules tumorales circulantes qui contiennent du ProNGF, et/ou des cellules tumorales circulantes qui sont capables de sécréter le ProNGF.

Ainsi, selon un mode de réalisation de l'invention, le fluide biologique est préalablement traité pour isoler les cellules tumorales circulantes contenues dans le dit fluide.

Par isoler les cellules tumorales circulantes, on entend obtenir une fraction cellulaire enrichie en cellules tumorales circulantes.

Le traitement du fluide pour isoler les cellules tumorales circulantes peut être effectué par tri cellulaire dans un cytomètre de flux, par enrichissement sur Ficoll, par enrichissement par billes magnétiques recouvertes d'anticorps spécifiques, ou par toute autre méthode d'enrichissement spécifique connue de l'homme du métier.

Dans le cas du sang ou de la moelle à titre de fluide biologique, les cellules tumorales circulantes peuvent être isolées grâce à une technique de séparation cellulaire sur Ficoll associée à une déplétion des cellules sanguines utilisant des anticorps anti-CD45 couplés à des billes magnétiques (Dynal Biotech ASA, Norvège).

La détection directe du ProNGF peut alors être effectuée directement à partir de cellules tumorales circulantes isolées du fluide biologique, par exemple par marquage immunocytochimique de ces cellules avec un anticorps anti-ProNGF, après avoir déposé les cellules tumorales circulantes sur une lame par cytospin. La détection directe du ProNGF peut également être effectuée directement dans les cellules tumorales circulantes en utilisant la méthode de cytométrie de flux telle que décrite dans Métézeau P, Ronot X, Le Noan-Merdrignac G, Ratinaud MH, La cytométrie en flux pour l'étude de la cellule normale ou pathologique (Tome I), Eds Medsi-MacGrrawhill.

Dans ces conditions, lesdites cellules circulantes peuvent être traitées dans des conditions permettant le blocage du ProNGF à l'intérieur desdites cellules. Un tel traitement est décrit par exemple dans Intracellular Flow Cytometry, Applied reagents and Techniques, pp 1-21, BD Phariningen.

La détection du ProNGF se fait alors après avoir rendu perméable la membrane des cellules pour faire rentrer les partenaires de liaison spécifique du ProNGF.

La détection directe du ProNGF à partir des cellules circulantes peut également être effectuée à l'aide du procédé décrit dans la demande de brevet WO03/076942 déposée par l'une des Demanderesses.

La détection directe du ProNGF dans les cellules tumorales peut encore être effectuée dans le milieu de culture desdites cellules après les avoir cultivées dans des conditions telles qu'elles sécrètent du ProNGF.

Ces conditions de culture sont des conditions classiques telles que 37°C sous atmosphère humide et à 5% de CO₂.

Lorsque l'échantillon biologique à tester est du tissu provenant de la biopsie de la tumeur ou des métastases du patient, ce qui constitue un mode de réalisation particulier de l'invention, la détection directe du ProNGF est effectuée directement sur les coupes obtenues, sans traitement préalable dudit tissu.

Un autre mode de détection de la présence du ProNGF consiste en la culture, en présence de l'échantillon biologique, de cellules sensibles au ProNGF, ce qui constitue un mode de réalisation particulier de l'invention.

Dans ce cas, la détection de la présence du ProNGF dans un échantillon biologique est mise en évidence par la réaction des cellules sensibles au ProNGF.

Par cellules sensibles au ProNGF, on entend toute cellule stimulée en présence de ProNGF (croissance, apotose, etc.).

A titre de cellules sensibles au ProNGF, on peut citer les cellules d'origine neuronale, telles par exemple que les cellules PC12 (Pedraza et al. Am. J Pathol. 2005, 166, 533-543).

L'échantillon biologique que l'on peut utiliser pour la détection de la présence du ProNGF par culture de cellules sensibles au ProNGF peut être tout échantillon tel que décrit précédemment.

Ainsi, l'échantillon biologique peut être constitué de fluide biologique, le cas échéant préalablement traité pour isoler les cellules tumorales circulantes, elles-mêmes pouvant ensuite être cultivées en conditions telles qu'elles sécrètent du ProNGF, comme décrit précédemment.

Le procédé de l'invention peut être utilisé tant pour le diagnostic précoce, que pour le dépistage, le suivi thérapeutique, le pronostic et le diagnostic des rechutes dans le cadre de cancer et en particulier des cancers du sein, de la thyroïde, du poumon ou de la prostate puisque seules les cellules cancéreuses produisent du ProNGF et que cette production est fonction du grade du cancer.

Ainsi, un autre objet de l'invention consiste en l'utilisation du procédé de l'invention dans le diagnostic précoce, le dépistage, le suivi thérapeutique, le pronostic et le diagnostic des rechutes dans le cadre du cancer et en particulier des cancers du sein, de la thyroïde, du poumon ou de la prostate.

Outre un rôle de marqueur tumoral, le ProNGF peut également avoir un rôle de cible thérapeutique. En effet, du fait de la capacité des cellules cancéreuses et en particulier des cellules des cancers du sein, de la thyroïde, du poumon ou de la prostate à produire du ProNGF, tandis que les cellules normales n'en produisent pas, la dissémination à distance des cellules du cancer du sein, de la thyroïde, du poumon ou de la prostate et l'invasion des cellules peuvent être bloquées par un inhibiteur du ProNGF susceptible de bloquer l'activité du ProNGF.

Par ailleurs le ProNGF, seul ou en association avec d'autres molécules, peut servir de cible thérapeutique pour l'adressage d'outils thérapeutiques. Les outils thérapeutiques peuvent être dans ce cas des nanoparticules activables, des cytotoxiques, ou toute autre molécule permettant la destruction des cellules cancéreuses.

Les inhibiteurs du ProNGF peuvent donc être utilisés comme médicaments. Ainsi, la présente invention a également pour objet l'utilisation d'un inhibiteur du ProNGF pour la préparation d'un médicament, en particulier dans le traitement du cancer et plus particulièrement le cancer du sein, de la thyroïde, du poumon ou de la prostate; caractérisée en ce que l'inhibiteur est un anticorps spécifique du ProNGF, les fractions d'anticorps spécifique du ProNGF, un analogue sous forme soluble d'un récepteur au ProNGF, un SiRNA d'un récepteur du ProNGF, un oligonucléotide anti-sens dudit récepteur, peptide dérivé du ProNGF ayant conservé les propriétés de liaison au récepteur du ProNGF ou un anticorps spécifique dirigé contre le récepteur du ProNGF.

Ledit médicament est utile pour bloquer la migration cellulaire ou l'invasion des cellules tumorales chez les patients atteints du cancer et plus particulièrement du cancer du sein, de la thyroïde, du poumon ou de la prostate.

On entend par migration cellulaire la dissémination à distance chez les patients atteints du cancer et plus particulièrement du cancer du sein, de la thyroïde, du poumon ou de la prostate (métastases) et on entend par invasion la pénétration locale des cellules cancéreuses.

Les compositions pharmaceutiques comprenant à titre de principe actif au moins un inhibiteur du ProNGF, éventuellement en association avec un excipient pharmaceutiquement acceptable, sont également incluses dans l'invention.

Les compositions pharmaceutiques utiles contre le cancer comprennent, à titre de principe actif, au moins un inhibiteur du ProNGF susceptible de bloquer migration cellulaire ou l'invasion.

Par inhibiteur du ProNGF, on entend les inhibiteurs directs du ProNGF, c'est-à-dire les inhibiteurs bloquant l'activité biologique de la protéine, tels que les partenaires de liaison du ProNGF, ainsi que les inhibiteurs des récepteurs au ProNGF ou tout inhibiteur des voies de signalisation du ProNGF, ainsi que toute molécule pouvant se lier spécifiquement au ProNGF, qu'il y ait blocage ou non de l'activité biologique.

Un exemple de récepteur au ProNGF exprimé par les cellules épithéliales du sein, tant tumorales que normales, comprend la Sortiline.

Les partenaires de liaison du ProNGF spécifiques appropriés à titre de principe actif sont notamment tels que définis précédemment dans les tests immunologiques et peuvent être tout autre partenaire connu de l'homme du métier capable de bloquer la migration ou invasion cellulaire. Selon un mode de réalisation particulier, le partenaire spécifique susceptible de bloquer la migration ou invasion de cellules de cancer du sein est un anticorps anti-ProNGF.

Un autre exemple d'inhibiteur direct du ProNGF comprend les analogues, sous forme soluble, de récepteurs au ProNGF, tel que les analogues de la Sortiline sous forme soluble, ce qui constitue également un mode de réalisation de l'invention.

Par inhibiteur de récepteurs au ProNGF exprimés par les cellules épithéliales du sein, tant tumorales que normales, on entend toutes molécules qui bloquent l'activité du ProNGF par exemple soit via ledit récepteur ou en bloquant sa production. Le blocage de l'activité du ProNGF via ledit récepteur peut être mis en oeuvre en empêchant la liaison du ProNGF audit récepteur, comme par exemple à l'aide d'un agent capable de se lier audit récepteur, prenant ainsi la place du ProNGF. Un exemple d'un tel inhibiteur comprend un peptide dérivé du ProNGF ayant conservé les propriétés de liaison au dit récepteur ou un anticorps dirigé contre le récepteur. On peut également bloquer l'activité du ProNGF en bloquant la production dudit récepteur à l'aide d'inhibiteurs de l'ARNm dudit récepteur ou du gène codant pour ledit récepteur. A titre d'inhibiteur de l'ARNm du récepteur, on peut utiliser un fragment synthétique de cet ARNm. En effet, la technologie du ARN interférence (Small Interference RNA ou SiRNA) est basée sur l'utilisation d'ùn oligonucléotide ARN double brin correspondant à une courte séquence de l'ARNm cellulaire à inhiber. Cet oligonucléotide en duplex (introduit ou non dans un plasmide), après entrée dans la cellule, est pris en charge par le système enzymatique Dicer/Risc, ce qui va entraîner la dégradation de l'ARNm cellulaire correspondant (Dykxhoorn D et al., 2003, Nature Reviews, vol. 4, p457-467). A titre d'inhibiteur du gène codant pour le récepteur, on peut citer un oligonucléotide anti-sens dudit récepteur. Cet oligonucléotide peut être facilement préparé par l'homme du métier (Lefrançois S et al. Biol. Proced. online. 2005, 7(1):17-25.

Selon un mode de réalisation particulier de l'invention, l'inhibiteur du ProNGF est un SiRNA du récepteur au ProNGF.

Par inhibiteur des voies de signalisation du ProNGF, on entend toute molécule qui bloque l'activité biologique du ProNGF telle que son activité sur la migration et l'invasion cellulaire.

Pour cibler l'action thérapeutique des différents inhibiteurs, ces derniers peuvent être mis en conditions telles qu'ils pénètrent spécifiquement dans les cellules d'intérêt, telles que les cellules tumorales, ce qui constitue un autre mode de réalisation de l'invention.

A cet effet, ils peuvent par exemple être fixés à un partenaire qui permet une telle pénétration, comme par exemple une molécule porteuse, un polymère tel que les polymères utilisés en thérapie génique ou bien un vecteur viral tel que les adénovirus et les poxvirus, également utilisés en thérapie génique.

Par exemple, dans le cadre du cancer du sein, la molécule porteuse peut être un anticorps anti-MUC1 ou un anticorps anti-cellule épithéliale, ou bien encore un anticorps anti-HER-2/neu. Dans le cadre du cancer de la thyroïde, la molécule porteuse peut être un anticorps anti-thyroglobuline ou un anticorps anti-cellule épithéliale. Dans le cancer de la prostate, la molécule porteuse peut être un anticorps anti PSA ou un anticorps anti-cellule épithéliale. Dans le cadre du cancer du poumon, la molécule proteuse peut être un anticorps anti-cellule épithéliale.

Les inhibiteurs des voies de signalisation du ProNGF) peuvent également être fixés à des agents anticancéreux. Par agent anticancéreux, on entend tout composé qui sera toxique pour la cellule cancéreuse. Par exemple, les partenaires du ProNGF peuvent être associés à des nanoparticules thérapeutiques, qui pourront permettre la destruction ciblée de la tumeur lorsqu'elle seront activées. Dans un autre procédé de l'invention, le partenaire du pro-NGF peut être couplé à un agent cytotoxique, ou à une molécule bloquant un processus de carcinogénèse.

De préférence, lorsque la composition pharmaceutique comprend à titre de principe actif un inhibiteur du ProNGF tel qu'un inhibiteur direct ou un inhibiteur des voies de signalisation du ProNGF, ces derniers sont mis en conditions telles qu'ils pénètrent spécifiquement dans les cellules tumorales d'intérêt, les inhibiteurs de l'ARNm du ProNGF et les inhibiteurs du gène codant pour le NGF ayant la capacité de pénétrer naturellement dans lesdites cellules.

La quantité et la nature de l'excipient peuvent être facilement déterminées par l'homme du métier. Elles sont choisies selon la forme pharmaceutique et le mode d'administration souhaités.

Les compositions pharmaceutiques de l'invention conviennent pour l'administration orale, sublinguale, sous-cutanée, intramusculaire, intratumorale, intraveineuse, topique, locale, intratrachéale, intranasale, transdermique, rectale, intraoculaire, intra-auriculaire, ledit principe actif pouvant être administré sous forme unitaire d'administration.

Les formes unitaires d'administration peuvent être par exemple des comprimés, des gélules, des granules, des poudres, des solutions ou suspensions orales injectables, des timbres transdermiques (« patch »), des formes d'administration sublinguale, buccale, intratrachéale, intraoculaire, intranasale, intra-auriculaire, par inhalation, des formes d'administration topique, transdermique, sous-cutanée, intramusculaire, intratumorale ou intraveineuse, des formes d'administration rectale ou des implants. Pour l'administration topique, on peut envisager des crèmes, gels, pommades, lotions ou collyres.

Ces formes galéniques sont préparées selon les méthodes usuelles des domaines considérés.

Lesdites formes unitaires sont dosées pour permettre une administration journalière de 0,001 à 10 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse du patient.

L'invention sera mieux comprise à l'aide des exemples suivants donnés à titre illustratif et non limitatif, ainsi qu'à l'aide des Tableaux 1 à 2, et des figures 1 à 10 annexées, sur lesquelles :
- la figure 1 représente une photographie de Western-blot, montrant la production de ProNGF par les cellules épithéliales mammaires cancéreuses (MCF-7, T47-D, BT-20 et MDA-MB-231) mais pas par des cellules normales (cellules CEMN), l'actine ayant été utilisée à titre de témoin positif,
- la figure 2 représente une photographie de Western-blot la présence de ProNGF dans les biopsies mammaires cancéreuses (ST-1 à -4) mais pas dans les biopsies normales (SS-1 à -4), l'actine ayant été utilisée à titre de témoin de normalisation,
- la figure 3 représente une photographie de Westem-blot montrant :
- figure 3A : la sécrétion du ProNGF par les cellules cancéreuses MCF-7 (piste milieu conditionné concentré), les 2 pistes de droite étant des pistes témoins,
- figure 3B : que l'unité de concentration/dessalage utilisé pour l'expérience de la figure 3A était capable de retenir le NGF,

- la figure 4 représente une photographie de Western-blot montrant la présence de ProNGF dans les sérums de souris injectées avec des cellules cancéreuses et son absence dans les sérums de souris normales,
- la figure 5 représente des graphes de détection par spectrométrie de masse de ProNGF dans le milieu conditionné par les MCF-7 (figures 5A, 5C et 5E) ou de ProNGF recombinant (figures 5B et 5D), donnant l'abondance relative en fonction de la masse,
- la figure 6 représente une photographie de Westem-blot montrant la présence de Sortiline dans les cellules épithéliales mammaires cancéreuses (MCF-7, T47-D, BT-20 et MDA-MB-231) et les cellules normales (cellules CEMN), l'actine ayant été utilisée à titre de témoin positif,

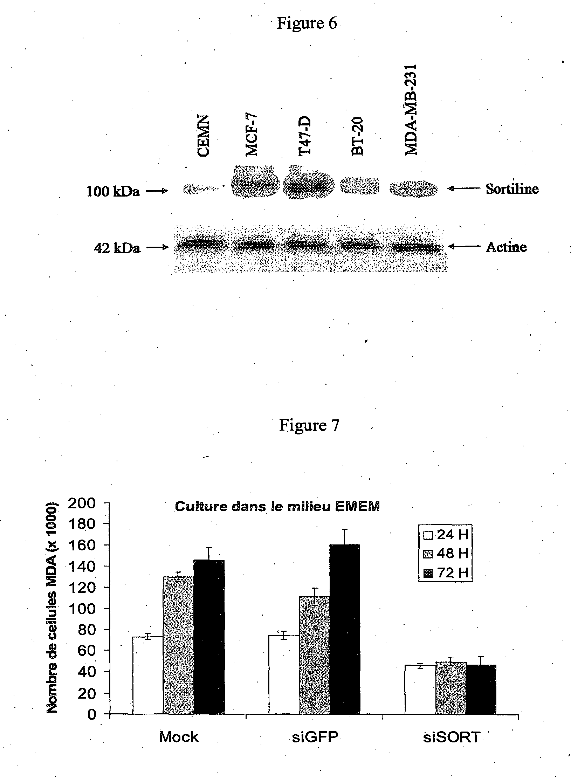

- la figure 7 représenté un graphe donnant le nombre de cellules MDA qui poussent dans le milieu de culture EMEM au bout de 24, 48 et 96 heures de culture, transfectées soit avec du milieu de culture seul (Mock), soit avec un ARN interférant contrôle négatif (siGFP, molécule d'ARN double brin, composée des séquences partiellement complémentaires SEQ ID No.1 et SEQ ID No. 2 dans lesquelles le nucléotide N représente la thymine T), soit avec un ARN interférant qui diminue l'expression de la Sortiline (siSORT, molécule d'ARN double brin, composée des séquences partiellement complémentaires SEQ ID No.3 et SEQ ID No. 4 dans lesquelles la base N représente la thymine T),

| | | |
|---|---|---|
| Séquence siGFP : | SEQ ID No. 1 | 5'-GCUGACCCUGAAGUUCAUCNN-3' |
| | SEQ ID No. 2 | 5'-GAUGAACUUCAGGGUCAGCNN-3' |
| Séquence siSORT : | SEQ ID No. 3 | 5'-GGUGGUGUUAACAGCAGAGNN-3' |
| | SEQ ID No. 4 | 5'-CUCUGCUGUUAACACCACCNN-3' |

- la figure 8 représente un graphe donnant le pourcentage de migration cellulaire à partir de cellules MCF-7 baignant soit dans du milieu de sevrage seul, soit dans du milieu de sevrage complémenté avec 200 ng/ml de ProNGF, soit dans du milieu de sevrage complémenté avec 200 ng/ml de ProNGF et 20 µM de galardine, soit dans du milieu de sevrage complémenté avec 20µM de galardine, soit dans du milieu de sevrage complémenté avec 200 ng/ml de NGF, et
- la figure 9 représente un graphe donnant l'index d'invasion à partir de cellules MCF-7 baignant soit dans du milieu de sevrage seul, soit dans du milieu de sevrage complémenté avec 200 ng/ml de ProNGF, soit dans du milieu de sevrage complémenté avec 200 ng/ml de ProNGF et 20 µM de galardine, soit dans du milieu de sevrage complémenté avec 20 µM de galardine, soit dans du milieu de sevrage complémenté avec 200 ng/ml de NGF.

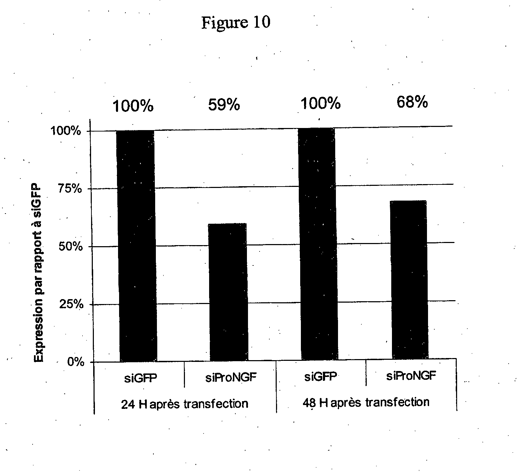

- la figure 10 tracée à partir des photographies de Western blot montre la diminution de l'expression du ProNGF dans les cellules MCF-7 suite à la transfection d'un ARN interférant ProNGF (siProNGF), au bout de 24 ou de 48 heures de culture. L'actine a été utilisée comme témoin positif pour valider l'équicharge. Les cellules MCF-7 ont été transfectées par des ARN interférants (siRNA) dirigés soit contre la GFP (siGFP, composé des séquences partiellement complémentaires SEQ ID No.1 et SEQ ID No. 2), soit contre le ProNGF (siProNGF, molécule d'ARN double brin, composée des séquences partiellement complémentaires SEQ ID No.5 et SEQ ID No. 6 dans lesquelles la base N représente la thymine T). La quantité relative de ProNGF détectée en blot a été évaluée avec le logiciel QuantityOne (Bio-Rad) rapportée à l'équicharge et présentée sous forme d'histogramme où 100% est attribué à la condition contrôle siGFP,

| | | |
|---|---|---|
| Séquence siGFF : | SEQ ID No. 1 | 5'-GCUGACCCUGAAGUUCAUCNN-3' |
| | SEQ ID No. 2 | 5'-GAUGAACUUCAGGGUCAGCNN-3' |
| Séquence siProNGF : | SEQ ID No. 5 | 5'-CAGUGUAUUCAAACAGUAUNN-3' |
| | SEQ ID No. 6 | 5'-GUACUGUUUGAAUACACUGNN-3' |

### Exemple 1 : Culture des cellules

### 1.1. Matériel

Les cellules utilisées sont des lignées établies de cellules de cancer du sein (BT-20, MCF-7, MDA-MB-231, T-47D) obtenues auprès de l'ATCC (American Type Culture Collection) et des CEMN (Cellules Epithéliales Mammaires Normales) en primoculture obtenues auprès de patientes ayant subies une mammoplastie au Centre Oscar Lambret de Lille. Les MCF-7, les MDA-MB-231 et les T-47D sont des cellules épithéliales qui proviennent d'effusions pleurales chez des patientes atteintes d'adénocarcinome ; les BT-20, quant à elles, proviennent d'un carcinome primaire. MCF-7 et T-47D sont dites « hormono-sensibles » car elles expriment des récepteurs aux oestrogènes, alors que les BT-20 et les MDA-MB-231 sont dites « hormono-insensibles » car elles n'en expriment pas.

Les biopsies proviennent de patientes ayant subi une mammoplastie de tissu non malin (Professeur Pellerin CHRU, Lille) ou une exérèse de tissu cancéreux (Docteur Laurent, Croisé Laroche, Marcq en Baroeul).

Les lames d'immunohistochimie (tissue array) contenant des spots de tissus tumoraux et sains mammaires proviennent de Biochain Inc. (Cat. No. T8235731). D'autres lames d'immunhistochimie contenant des tissus issus de divers organes (tissus cancéreux et tissus sain correspondant) proviennent de Superbio Chips (Cat. No. MA (tissu cancer), MAN (tissu sain correspondant) et AA (organes sains divers)).Des souris femelles SCID (Severe Combined ImmunoDeficiency) de 6 semaines ont été obtenues auprès de Iffa Credo (France) et acclimatées pendant au moins 2 semaines. Ces souris sont élevées à 20-22°C en maintenant une alternance jour/nuit de 12 h (lumière de 6H00 à 18H00) et nourries *ad libitum* dans le respect des directives fixées par l'Institutional Animal Care.

Le ProNGF utilisé est du recombinant humain commercialisé par SCIL proteins (Allemagne). L'EGF, l'HGF et le NGF recombinant ont été obtenus chez R&D systems (France). Le cortisol, l'insuline, le DMSO (DiMethyl SulfOxide), la toxine cholérique, la transferrine, l'analogue du céramide C2 (N-acetyl-D-sphingosine), le Hoechst 33258 (BisBenzimide), ont été obtenus auprès de Sigma (France). EMEM (Eagle's Minimum Essential Medium), DMEM/F12 (Dulbecco's Modified Eagle's Medium), trypsine-EDTA (Ethylène-Diamine-Tetra-Acétate), HEPES (acide N-2-Hydroxy-Ethyl-Piperazine-N'-2'-Ethan-Sulfonique), L-glutamine, acides aminés non essentiels, pénicilline/streptomycine, gentamycine sont commercialisés par Cambrex (France). Le SVF (Sérum de Veau Foetal) est commercialisé par Gibco Invitrogen Corporation (France). Les différents plastiques utilisés : cryotubes 1 mL, tubes à centrifuger de 15 et 50 mL, boîtes Pétri de 100 mm de diamètre, boîtes 75 cm², boîtes 175 cm² et plaques 6 puits (puits de 35 mm de diamètre) sont commercialisés par Starstedt (Allemagne). Les transwell® sont obtenus auprès de Costar (France). La fibronectine est obtenue chez Becton Dickinson (France). Le glycergel est commercialisé par Dako (France). La galardine est obtenue auprès de Tebu-Bio (France). Le collagène de type I est vendu par Upstate (Etats-Unis d'Amérique).

### 1.2. Décongélation et entretien des cellules

Les cryotubes de cellules sont sortis de l'azote liquide et décongelés 2 minutes à 37°C. La suspension est ensuite récupérée et transférée dans un tube à centrifuger de 50 mL contenant 19 mL de MEM (Minimum Essential Medium). L'ensemble est centrifugé à 200 g (15 minutes) pour enlever les traces de DMSO (DiMethyl SulfOxide). Le culot cellulaire est récupéré puis repris avec 10 mL de milieu complet (composition ci-dessous) et transféré dans une boîte 75 cm². Le milieu est changé après 24 heures et 24 heures plus tard les cellules sont passées. Les cellules sont ensuite entretenues pendant 2 semaines avant toute expérimentation.

Les lignées cancéreuses sont entretenues dans des boîtes de 75 cm² sur lesquelles elles adhèrent et poussent en monocouche dans un milieu complet : EMEM supplémenté de 10% de SVF, 20 mM d'HEPES, 2 g/L de bicarbonate de sodium, 2 mM de L-glutamine, 1 % d'acides-aminés non essentiels, 40 u/mL de pénicilline/streptomycine et de 50 µg/mL de gentamycine. Les CEMN sont entretenues dans des boîtes de 75 cm² sur lesquelles elles adhèrent et poussent en monocouche dans du milieu complet : DMEM/F12 supplémenté de 5% de SVF, 10 u/mL d'insuline, 5 µM de cortisol, 2 ng/mL d'EGF, 0,01 ng/mL de toxine cholérique, 100 µg/mL de pénicilline et 45 µg/mL de gentamycine.

Les cellules sont cultivées en atmosphère humide à 37°C et à 5% de CO₂. Le milieu d'entretien est changé tous les 2 jours et les cellules sont passées à confluence.

### Exemple 2 : Détection du ProNGF

### 2.1. Méthodologie

### Lyse des cellules et des biopsies mammaires : extraction protéique

Les cellules sont ensemencées dans 3 boîtes Pétri de diamètre 100 mm, puis à 95% de confluence les cellules sont rincées 2 fois sur glace au PBS (Phosphate Buffered Saline) : KCl 107 mM, KH₂PO₄ 59 mM, NaCl 137 mM, Na₂HPO₄ 56 mM, lysées avec 100 µL/boîte d' un tampon de lyse contenant : 50 mM de Tris-HCl pH 7,5, 150 mM de NaCl, 1% de Nonidet P-40, 1% de SDS (Sodium Dodecyl Sulfate), et des inhibiteurs, d'activité protéasique : 1 mM de Phenyl-methyl-sulfonide-fluoride, 1 mM d'orthovanadate, 1 mM de Na₄P₂O₇, 10 µg/mL de leupeptine, 10 µg/mL d'aprotinine. Les boîtes sont congelées 12 heures puis raclées et les lysats sont poolés, chauffés 5 min à 100°C et centrifugés à 10 000 g (10 min à 4°C) et le surnageant est récupéré.

Les biopsies sont pesées et placées avec 10 fois leur volume de tampon de lyse (précédemment décrit) sur roue (1 heure à 4°C). Elles sont ensuite broyées, sur glace, au dounce, congelées (20 minutes à -80°C) et décongelées sur glace. Enfin, elles sont centrifugées à 13 000 g (10 minutes à 4°C), une galette lipidique est éliminée et le surnageant est récupéré.

Les surnageants sont dosés par la méthode à l'acide Bicinchoninique comparé à une gamme de sérumalbumine bovine puis, aliquoté en 50 µL avant congélation.

### Obtention de milieu conditionné et de sérum

Les MCF-7 sont ensemencées dans du milieu complet dans des boîtes 175 cm². A 90% de confluence, ces boîtes sont rincées 2 fois dans du milieu de sevrage puis les cellules sont sevrées 24 heures avec 14 mL de milieu de sevrage. Après ce délai, le milieu conditionné par les cellules est récupéré et centrifugé à 1 000 g (15 minutes à 4°C). Le surnageant est ensuite directement utilisé ou congelé à -20°C. Des unités de concentration/dessalage avec un seuil de coupure de 10 kDa (Centricon Plus 20 Millipore, France) sont chargées chacune avec 14 mL de milieu conditionné par les MCF-7 puis centrifugées à 4 000 g (15 minutes à 4°C). Ces mêmes unités ont été rechargées encore 3 fois avec 14 mL de milieu conditionné (ce qui aboutit à une concentration d'un facteur 4 du milieu de départ). Puis les unités sont dessalées à l'eau de qualité mQ (18,2 osm) par chargement de 14 mL et centrifugation à 4 000 g (15 minutes à 4°C), ceci 2 fois selon les recommandations du fabricant. Enfin le concentrat est récupéré par inversion de l'unité et centrifugation à 1 000 g (4 minutes à 4°C) ; 250 µL sont récupérés. Le milieu conditionné concentré est ensuite aliquoté en 50 µL et congelé à -20°C. On aboutit alors à un facteur de concentration de 4x14 000/250 soit 224 fois.

Les MDA-MB-231 (3x10⁶) sont resuspendues dans du PBS, puis injectées de manière sous-cutanée dans les flancs de souris SCID agées de 8 semaines. Le volume de la tumeur est mesuré après deux semaines tous les deux jours. Après 7 semaines, les souris sont anesthésiées à l'éther, puis le sang est collecté par prélèvement intracardiaque, avant le sacrifice des animaux. Le sang est laissé une nuit à 4°C pour permettre la coagulation, le sérum étant récupéré après centrifugation, dosé et congelé à -204°C.

### SDS-PAGE

Les aliquots (50 µL) sont décongelés avant utilisation, repris avec 12,5 µL de tampon Laëmmli 5x : 5% SDS, 5% β-mercapto-éthanol, 50% glycérol, 50 mM Tris pH 6,8, 0,3% bleu de bromophénol. Les protéines sont déposées dans les puits d'un gel de polyacrilamide de 12,5%. Après migration (sous 30 mA, pendant 5 heures) et transfert sur membrane de nitrocellulose (sous 200 mA, pendant 1 heure), la qualité du transfert est appréciée par une coloration au rouge Ponceau.

### Immunodétection

Les membranes sont saturées par de la sérum-albumine bovine à 4% dans une solution de TBS (Tris Buffered Saline) 0,1% Tween20 (17.54 g NaCl, 2.42 g Tris, 2 mL tween20, QSP 2 L, pH ajusté à 7.4) à température ambiante pendant 2 heures. Après saturation, les membranes sont incubées pour la nuit à 4°C avec les différents anticorps primaires : anti-ProNGF (AB9040 de chez Chemicon) au 1 : 2 000, anti-NGF (SC-548 de chez Sigma) au 1 : 2 000 ou anti-Actine (A-2066 de chez Sigma) au 1 : 5 000 dans la solution de saturation. Après rinçages au TBS 0,1% Tween20, les membranes sont incubées 1 heure à température ambiante avec un anticorps secondaire au 1 : 20 000, anti-lapin (A-1949 de chez Sigma, France) dans la solution de saturation, couplé à une peroxydase de raifort. Après rinçage, la révélation est réalisée avec le système ECL (Pierce Interchim, France) suivant les données d'utilisation recommandées.

### Immunohistochimie

Dans un premier temps, les lames de tissue array sont déparaffinées. Pour cela, elles sont incubées successivement dans les bains suivants pendant 10 minutes : methycyclohexane (2 fois), éthanol 100%, éthanol 95%, éthanol 70% et eau. Les lames sont ensuite rincées au TBS 0,1% Tween 20 (TBS-T), pendant 10 min, sous agitation. Les antigènes sont réactivés dans le tampon citrate 10 mM pH6, en chauffant jusqu'à 90°C pendant 40 min, puis en laissant refroidir à température ambiante pendant 30 min. Les peroxydases endogènes sont inhibées par incubation dans du TBS-T contenant 3% de H₂O₂, pendant 5 min. Les lames sont ensuite saturées par 3% de BSA en TBS-T, pendant 1h à 37°C, en chambre humide. Puis, les lames sont incubées pendant 2h avec l'anticorps primaire anti-ProNGF (AB9040 de Chemicon) dilué au 1/200 dans du TBS-T contenant 3% de BSA (incubation à 37°C en chambre humide). Après 3 lavages de 10 min au TBS-T, les lames sont incubées 2h à 37°C en chambre humide avec l'anticorps secondaire anti-lapin couplé à la peroxydase de raifort (Cat. No. 711-035-152 Jacksori Immunoresearch) dilué au 1/400 dans la solution de saturation. Les lames sont lavées 3 fois 10 minutes dans du TBS-T, puis encore 3 fois 10 min dans du PBS. La révélation est réalisée avec le substrat Sigma Fast (Cat. No. D-4168, Sigma-Aldrich) pendant 5 min. La coloration est arrêtée par lavage dans le PBS. On effectue une contre-coloration à l'hématoxyline de Harris (Cat. No. MHS16, Sigma-Aldrich) pendant 30 sec. Après lavages à l'eau et au PBS, les lames sont montées pour observation au microscope.

### Spectrométrie de masse (SM)

Le milieu conditionné par les MCF-7 est injecté sur une nano colonne LC (Liquid Chromatography) C4 (Dionex, France) qui permet de séparer les protéines entières en fonction d'un gradient d'hydrophobicité croissant. Ainsi les protéines les plus hydrophobes sont éluées en dernier. Une fois séparées, les protéines entières sont ionisées par nanoESI (ElectroSpray Ionization), puis elles sont analysées dans une trappe ionique (Station LCQ Deca XP⁺™ de Thermo Electron) en fonction de leur masse (m) et de leur charge (z). Nous avons utilisé la technique du SIM (Selected Ion Monitoring) scan qui permet de scanner uniquement certains ions d'intérêt. Ces ions multichargés, caractéristiques du ProNGF recombinant, ont été déterminés au préalable en nanoLC-nanoESI/SM. Une fois détectés dans le milieu conditionné à l'aide du SIM du ProNGF, le spectre de masse multichargé correspondant est déconvolué pour retrouver la masse de la protéine qui a généré ce spectre.

### 2.2. Résultats

### Détection à partir des cellules épithéliales

50 µg d'extraits protéiques totaux de cellules épithéliales mammaires sont déposés sur gel pour une SDS-PAGE. Après transfert sur membrane de nitrocellulose, une immunodétection avec l'anticorps AB9040 et l'anticorps A-2066 (contrôle d'équicharge) est réalisée. CEMN : cellules épithéliales mammaires normales, BT-20, MCF-7, MDA-MB-231, T-47D sont des lignées de cellules épithéliales mammaires cancéreuses.

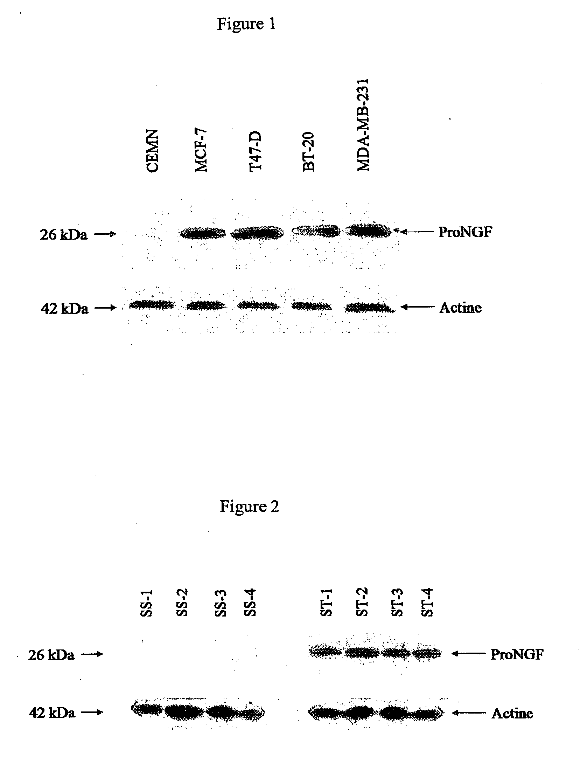

Les résultats sont indiqués sur la figure 1 qui représente une photographie de Western-blot montrant la production de ProNGF par les cellules épithéliales mammaires cancéreuses (MCF-7, T47-D, BT-20 et MDA-MB-231) mais pas par des cellules normales (cellules CEMN), l'actine ayant été utilisée à titre de témoin positif.

### Détection à partir de biopsies mammaires par Western blot

50 µg d'extraits protéiques de biopsies sont déposés sur gel pour une SDS-PAGE. Après transfert sur membrane de nitrocellulose, une immunodétection avec l'anticorps AB9040 et l'anticorps A-2066 (contrôle d'équicharge) est réalisée. SS-x : biopsie de sein sain numéro x, ST-x : biopsie de sein tumoral numéro x.

Les résultats sont indiqués sur la figure 2 qui représente une photographie de Western-blot la présence de ProNGF dans les biopsies mammaires cancéreuses (ST-1 à -4) mais pas dans les biopsies normales (SS-1 à -4), l'actine ayant été utilisée à titre de témoin positif.

### Détection à partir de biopsies mammaires par immunohistochimie

Des lames de tissue array ont été utilisées pour cribler un grand nombre de prélèvements. Il s'agit de biopsies mammaires spottées sur lames. Les tissus arrays utilisés ici contiennent 60 spots de biopsies correspondant à 1 contrôle, 4 donneurs sains et 25 patients atteints de cancer du sein, en dupliquât. Les caractéristiques des patients ainsi que l'intensité de l'immunomarquage par l'anticorps anti-ProNGF sont récapitulées dans le Tableau 1. Dans les biopsies mammaires issues de donneurs sains, un faible marquage est présent (intensité + chez 4/4 patients) alors que le signal est bien plus fort dans les tissus mammaires cancéreux (intensité ++ /+++ chez 20/25 patients). De plus le profil de marquage est différent entre les deux types de tissus : dans le tissu cancéreux le marquage est essentiellement épithélial alors que dans les biopsies saines, il ne l'est pas.

**Tableau 1. Caractéristiques des biopsies mammaires présentes sur le tissue array cancer du sein et intensité du marquage immunohistochimique par l'anticorps anti-ProNGF.**

| Identifiant | Diagnostic | Niveau de différenciation | Classification TNM | Stade | Intensité du marquage |
|---|---|---|---|---|---|
| HT00031 | Contrôle | | | | 0 |
| HT00500 | Sein normal | | | | + |
| HT00501 | Sein normal | | | | + (pas d'épithélium) |
| HT00502 | Sein normal | | | | + (pas d'épithélium) |
| HT00503 | Sein normal | | | | + (pas d'épithélium) |
| HT00509 | Carcinome canalaire invasif | Moyen | T2N0M0 | IIB | 0 |
| HT00510 | Carcinome canalaire invasif | Moyen | T2N0M0 | IIB | ++ |
| HT00511 | Carcinome lobulaire invasif | Fort | T2N11M0 | IIIB | ++ |
| HT00512 | Carcinome lobulaire invasif | Fort | T2N17M1 | IV | +++ |
| HT00513 | Carcinome lobulaire invasif | Moyen | T2N1M0 | IIB | + |
| HT00520 | Carcinome canalaire invasif | Moyen | T2N1M0 | IIB | 0 |
| HT00521 | Carcinome canalaire invasif | Moyen | T2N0M0 | IIB | ++ |
| HT00522 | Carcinomecanalaireinvasif | Moyen | T2N1M1 | IV | +++ |
| HT00523 | Carcinome canalaire invasif | Moyen | T2N3M1 | IV | ++ |
| HT00524 | Carcinome canalaire invasif | Moyen | T2N4M1 | IV | + |
| HT00531 | Fibroadénome | Inconnu | T2N0M0 | IIB | 0 |
| HT00532 | Carcinome canalaire invasif | Moyen | T2N2M0 | IIIA | ++ |
| HT00533 | Carcinome canalaire invasif | Faible | T2N0M0 | IIB | +++ |
| HT00534 | Carcinome canalaire invasif | Faible | T2N1M0 | IIB | ++ |
| HT00535 | Carcinome canalaire invasif | Moyen-Faible | T2N1M0 | IIB | +++ |
| HT00542 | Carcinome canalaire invasif | Moyen | T2N0M0 | IIB | 0 |
| HT00543 | Carcinome canalaire invasif | Moyen | T2N0M0 | IIB | +++ |
| HT00544 | Carcinome canalaire invasif | Moyen | T2N0M0 | IIB | +++ |
| HT00545 | Carcinome canalaire invasif | Moyen | T2N0M0 | IIB | +++ |
| HT00546 | Carcinome canalaire invasif | Moyen | T2N0M0 | IIB | +++ |
| HT00553 | Carcinome canalaire invasif | Moyen | T2N3M0 | IIIB | 0 |
| HT00554 | Carcinome canalaire invasif | Faible | T2N1M0 | IIB | +++ |
| HT00555 | Carcinome canalaire invasif | Moyen | T2N0M0 | IIB | +++ |
| HT00556 | Carcinome canalaire invasif | Moyen | T2N0M0 | IIB | +++ |
| HT00557 | Carcinome canalaire invasif | Moyen | T2N0M0 | IIB | +++ |

### Détection à partir de biopsies pulmonaires et thyroïdiennes par immunohistochimie

D'autres lames de tissue array ont été utilisées pour analyser l'expression du ProNGF dans d'autres types de cancers que le cancer du sein. Nous avons criblé 9 patients pour chaque cancer et analysé en parallèle le tissu tumoral et le tissu sain prélevé chez le même patient à proximité de la tumeur. Ainsi nous avons pu mettre en évidence une surexpression du ProNGF dans le cas de cancer du poumon et de la thyroïde. Les caractéristiques des patients ainsi que l'intensité de l'immunomarquage par l'anticorps anti-ProNGF sont récapitulées dans le Tableau 2. Pour le cancer du poumon comme celui de la thyroïde, le marquage est bien plus fort dans le tissu tumoral que le tissu sain chez 7 patients sur 9. Pour les autres patients (2/9 pour chaque cancer), les signaux gardent la même intensité dans les tissus tumoraux et sains. Rappelons que notre technique d'analyse n'est que semi-quantitative et qu'elle ne peut pas mettre en évidence de faibles différences d'expression.

**Tableau 2. Caractéristiques des biopsies pulmonaires et thyroïdiennes présentes sur le tissue array et intensité du marquage immunohistochimique par l'anticorps anti-ProNGF.**

| Identifiant | Diagnostic | Niveau de différenciation | Classification TNM | Stade | Intensité du marquage | |
|---|---|---|---|---|---|---|
| | | | | | Tissu Cancéreux | Tissu Normal |
| Cancers du poumon | | | | | | |
| P1 | Adénocarcinome | Fort | T2N0M0 | IB | +++ | ++ |
| P2 | Carcinome à grandes cellules | | T2N0M0 | IB | +++ | ++ |
| P3 | Cancer épidermoïde | Moyen | T2N0M0 | IB | +/++ | ++ |
| P4 | Cancer épidermoïde | Moyen | T2N1M0 | IIB | ++ | ++ |
| P5 | Cancer épidermoïde | Moyen | T4N0M0 | IIIB | ++ | + |
| P6 | Adénocarcinome | Fort | T2N0M0 | IB | ++ | + |
| P7 | Cancer épidermoïde | Moyen | T2N1M0 | IIB | +++ | + |
| P8 | Cancer épidermoïde | Moyen | T3N2M0 | IIIA | +++ | + |
| P9 | Cancer épidermoïde | Fort | T2N1M0 | IIB | +++ | + |
| | | | | | | |
| Cancers de la thyroïde | | | | | | |
| T1 | Carcinome papillaire | | T2N0M0 | I | ++ | + |
| T2 | Carcinome papillaire | | T2N0M0 | I | ++ | ++ |
| T3 | Carcinome papillaire | | T2N0M0 | I | +++ | +++ |
| T4 | Carcinome papillaire | | T2N0M0 | II | +++ | +/++ |
| T5 | Carcinome papillaire | | T2N0M0 | II | ++/+++ | + |
| T6 | Carcinome papillaire | | T3N1aM0 | III | +++ | + |
| T7 | Carcinome papillaire | | T1N0M0 | I | +++ | + |
| T8 | Carcinome papillaire | | T2N1bM0 | IVA | ++/+++ | + |
| T9 | Carcinome papillaire | | T3N0M0 | III | ++/+++ | + |

### Sécrétion du ProNGF

Afin de déterminer si le ProNGF est sécrété, nous avons concentré du milieu conditionné par des MCF-7. Pour ce faire, 100 µL de milieu conditionné concentré 224 fois sont soumis, après électrophorèse et transfert, à une immunodétection avec l'anticorps SC-548. En contrôles, le NGF et le ProNGF recombinants ont été analysés.

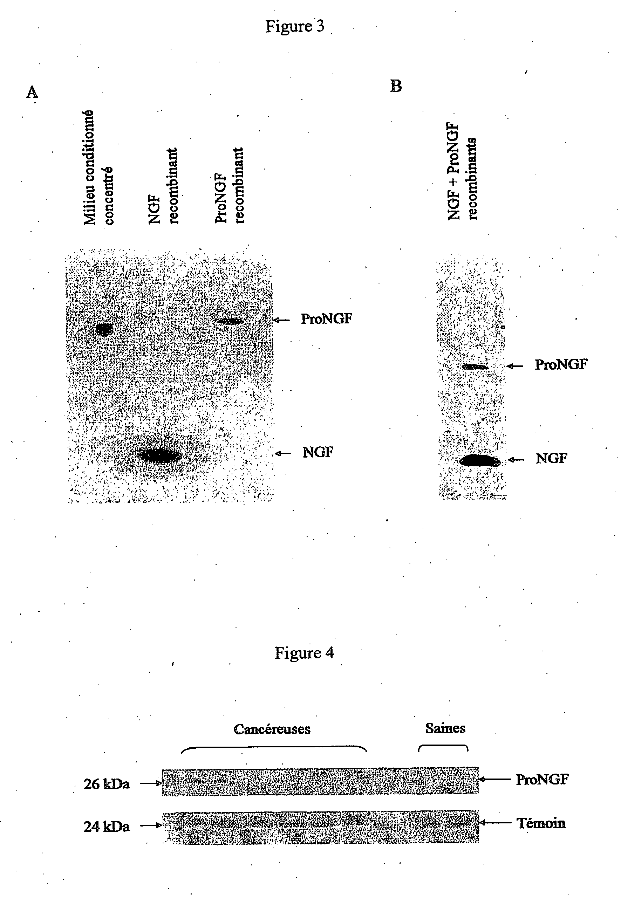

Les résultats sont indiqués sur la figure 3A qui représente une photographie de Western-blot montrant la sécrétion du ProNGF par les cellules cancéreuses MCF-7 et l'absence de sécrétion de NGF (piste milieu conditionné concentré), les 2 pistes de droite étant des pistes témoins. On peut noter qu'on n'observe ici aucune sécrétion de NGF parce que l'expérience est arrêté trop tôt pour observer du NGF du fait de l'abondance de ProNGF. En poursuivant l'expérience, on observe également une bande pour le NGF. Cela met en évidence que, non seulement le ProNGF est un marqueur tumoral, mais il est également sécrété en grande quantité.

Pour vérifier que l'unité de concentration/dessalage utilisée, avec un seuil de coupure de 10 kDa, était capable de retenir le NGF (13,6 kDa), nous avons concentré un mélange de ProNGF et de NGF recombinants dans les mêmes conditions expérimentales : 20 ng de ProNGF et 20 ng de NGF ont été ajoutés à du milieu non conditionné puis ont subi les mêmes conditions que le milieu conditionné.

Les résultats (Fig. 3.B) prouvent que l'unité de concentration/dessalage est capable de retenir le NGF et le ProNGF.

### Détection dans le sérum

100 µg d'extraits protéiques de sérums de souris sont déposés sur gel pour une SDS-PAGE. Après transfert sur membrane de nitrocellulose, une immunodétection avec l'anticorps AB9040 est réalisée. En contrôle d'équicharge une bande visible à 24 kDa sur la membrane après coloration au Ponceau est présentée. Cancéreuses : 6 sérums de souris injectées avec des MDA-MB-231, Saines : 2 sérums de souris injectée avec du PBS.

Les résultats sont indiqués sur la figure 4 qui montre qu'une bande immunoréactive pour le ProNGF apparaît à 26 kDa (poids du ProNGF) dans 5 sérums de souris injectées avec des cellules cancéreuses sur 6 ; et aucune bande n'a été détectée dans les sérums des animaux contrôles.

### Détection par spectrométrie de masse

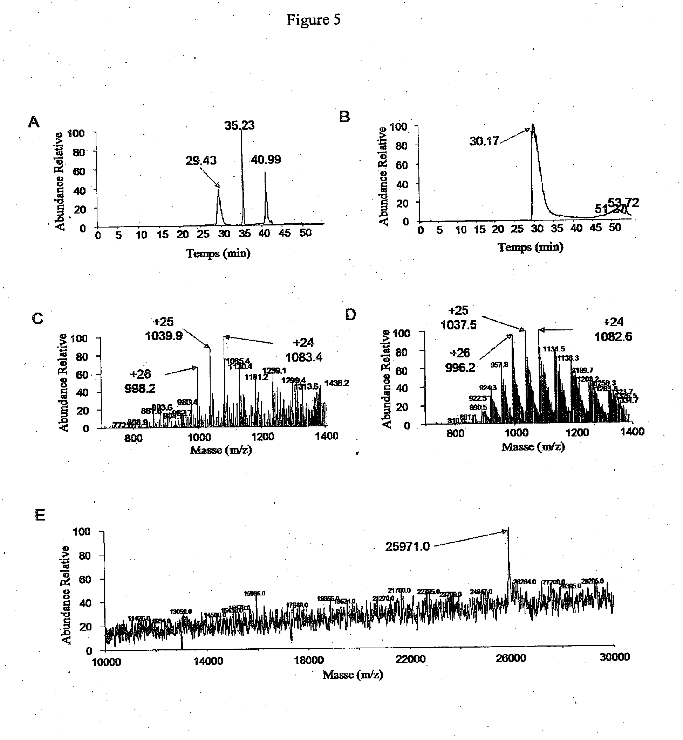

Nous avons procédé comme indiqué précédemment. Les résultats sont indiqués sur la figure 5 qui représente des graphes de détection par spectrométrie de masse de ProNGF dans le milieu conditionné par les MCF-7 (figures 5A, 5C et 5E) ou de ProNGF recombinant (figures 5B et 5D), donnant l'abondance relative en fonction de la masse. Les figures 5A et B correspondent à la fraction protéique de la nanoLC répondant au SIM d'au moins un des trois ions caractéristiques du ProNGF. La figure 5C correspond au spectre de masse de la protéine éluée à 29,43 min en A. La figure 5D correspond au spectre de masse du ProNGF recombinant avec ses 3 ions caractéristiques en rouge. Enfin, la figure 5E montre la déconvolution du spectre de masse de la protéine éluée à 29,43 min en A.

Ces résultats montrent qu'il apparaît dans le milieu conditionné par les MCF-7, une protéine répondant au SIM (Selected Ion Monitoring) du ProNGF, qui est éluée en même temps (29,43 min, Fig.5.A) que le ProNGF recombinant (30,17 min, Fig. 5.B). Cette protéine, une fois ionisée, génère les mêmes ions multichargés (998,2, 1039,9, 1083,4, Fig. 5.C) que le ProNGF recombinant (996,2, 1037,5, 1082,6, Fig. 5.D). De plus, le calcul par déconvolution de la masse de la protéine donne une valeur de 25 971 Da (Fig. 5.E). Ainsi, ces résultats montrent que d'une part que nous avons bien identifié du ProNGF dans le milieu conditionné par les MCF-7, et que d'autre part la détection de ProNGF par spectrométrie de masse est possible.

### Exemple 3 : Le ProNGF cible thérapeutique

### 3.1. Méthodologie

### Expression de la Sortiline, récepteur du ProNGF

Les méthodes de réalisation de l'extraction des protéines sont identiques à celles décrites pour le ProNGF (voir point 2.1 ci-dessus).

Pour le Western-blot les membranes sont saturées par de la sérum-albumine bovine à 4% dans une solution de TBS (Tris Buffered Saline) 0,1% Tween20 (17.54 g NaCl, 2.42 g Tris, 2 mL tween20, QSP 2 L, pH ajusté à 7.4) à température ambiante pendant 2 heures. Après saturation, les membranes sont incubées pour la nuit à 4°C avec les différents anticorps primaires : anti-Sortiline (BD612101 de chez BD Biosciences) au 1 : 2000, anti-Actine (A-2066 de chez Sigma) au 1 : 5 000 dans la solution de saturation. Après rinçages au TBS 0,1% Tween20, les membranes sont incubées 1 heure à température ambiante avec un anticorps secondaire au 1 : 20 000 anti-lapin (A-1949 de chez Sigma, France), dans la solution de saturation, couplé à une peroxydase de raifort. Après rinçage, la révélation est réalisée avec le système ECL (Pierce Interchim, France) suivant les données d'utilisation recommandées.

### Transfection d'ARN interférant (siRNA)

Les expériences de transfection d'ARN interférant (siRNA) ont été réalisées avec le kit Cell Line Nucleofector d'Amaxa et l'appareil d'électroporation correspondant. Ce système permet d'avoir une grande efficacité de transfert de gène vers le noyau (nucléofection).

Les cellules MDA-MB-231 sont passées 2 jours avant la transfection, pour atteindre 80% de confluence au moment de l'expérience. Les cellules à transfecter sont trypsinées puis comptées. Le culot cellulaire (1 million de cellules) est repris dans 100 µl de solution Nucleofector Kit V et 3 µg de siRNA sont ajoutés. Cette suspension cellulaire est transférée dans une cuve d'électroporation, placée dans l'appareil Amaxa et électroporée selon le programme X-13. Les cellules sont ensuite remises en culture dans un puits de plaque 6 puits pour 24-72h. La prolifération des cellules est suivie au cours du temps et elles sont comptées à des intervalles de temps réguliers. Un Western blot anti-Sortiline est réalisé sur les lysats récupérés à différents temps, selon le protocole décrit dans le paragraphe précédent, « Expression de la Sortiline ».

Les cellules MCF-7 sont passées 3-4 jours avant la transfection, pour atteindre 50% de confluence au moment de l'expérience. Deux millions de cellules sont repris dans 100 µl de solution Nucleofector Kit V et 3 µg de siRNA sont ajoutés. Le programme de transfection utilisé sur le Nucleofector est le E-14. Un Western blot anti-ProNGF est réalisé sur les lysats récupérés après 24 heures ou après 48 heures selon le protocole décrit au point 2.1. La quantité relative de ProNGF détectée en blot est évaluée avec le logiciel QuantityOne (Bio-Rad) rapportée à l'équicharge (actine) et présentée sous forme d'histogramme où 100% est attribué à la condition contrôle siGFP.

### Mesures d'activités biologiques du ProNGF

On a utilisé un milieu dit de « sevrage » qui désigne un milieu identique au milieu complet mais sans sérum et supplémenté de 2 µg/mL de fibronectine et de 30 µg/mL de transferrine. Pour les différentes mesures d'activités biologiques, c'est à ce milieu de sevrage que sont ajoutées les différentes molécules à tester (ProNGF, NGF et galardine). Lors des traitements, les milieux sont renouvelés toutes les 24 heures.

### Migration et invasion

Les tests de migration et d'invasion sont réalisés comme précédemment décrits par Bracke et al. (1999, J Natl Cancer Inst, 91 : 354-359). Les protocoles d'utilisation des chambres de Boyden et d'invasion en gel de collagène sont décrits ci-dessous.

### Chambre de Boyden (transwell®)

Des plaques 12 puits contenant des transwell® avec un diamètre de pores de 12 µm sont équilibrées avec du milieu de sevrage et placées à 37°C sous 5% de CO₂ pendant 2 heures. Puis le milieu est aspiré et remplacé dans la chambre inférieure par du milieu de sevrage avec les différentes molécules à tester, alors que dans la chambre supérieure 40 000 cellules sont ensemencées dans du milieu de sevrage seul. Après 24 heures, les transwell® sont rincés au PBS, la face supérieure est raclée puis un marquage au Hoechst est réalisé (comme décrit pour le test de survie), de façon à visualiser les cellules ayant traversé la membrane. Les transwell® sont montés entre lame et lamelle au moyen d'une goutte de glycergel chauffé à 55°C, puis les lames sont conservées à 4°C à l'obscurité jusqu'au comptage. Chaque condition est réalisée en duplicate, un minimum de 40 champs est compté pour chaque condition. Les données représentent la moyenne pondérée par les écart-types des comptages sur ces 40 champs. Elles sont présentées en pourcentage de migration où 100% de migration sont attribués à un témoin HGF à 50 ng/mL.

### Test d'invasion en gel de collagène

Le gel de collagène de type 1 est préparé de la façon suivante : 2,1 mL de collagène de Type 1, 0,8 mL de EMEM (10x), 4,6 mL de PBS, 0,8 mL de NaHCO₃ à 0,25 M, 0,15 mL de NaOH 1 M. 1,25 mL sont déposés dans les puits d'une plaque 6 puits. Une fois le gel solidifié, 100 000 cellules sont ensemencées avec du milieu de sevrage et les différentes molécules à tester pour 24 heures. Les cellules sont ensuite comptées et l'index d'invasion est déterminé (nombre de cellules en profondeur rapporté au nombre de cellules à la surface). Chaque condition est réalisée en triplicate et un minimum de 45 champs est compté par condition.

### 3.2. Résultats

### Expression de la Sortiline, récepteur du ProNGF

50 µg d'extraits protéiques totaux de cellules épithéliales mammaires sont déposés sur gel pour une SDS-PAGE. Après transfert sur membrane de nitrocellulose, une immunodétection avec l'anticorps BD612101 et l'anticorps A-2066 (contrôle d'équicharge) est réalisée. CEMN : cellules épithéliales mammaires normales, BT-20, MCF-7, MDA-MB-231, T-47D sont des lignées de cellules épithéliales mammaires cancéreuses.

Les résultats sont donnés sur la figure 6 qui représente une photographie de Western-blot montrant la présence de Sortiline dans les cellules épithéliales mammaires cancéreuses (MCF-7, T47-D, BT-20 et MDA-MB-231) et les cellules normales (cellules CEMN), l'actine ayant été utilisée à titre de témoin positif. La Sortiline apparaît donc dans toutes les cellules. En revanche, cette bande apparaît plus intense dans les cellules cancéreuses que dans les cellules normales, alors que le témoin de charge, l'actine, ne varie pas. On peut donc penser qu'il y a moins de Sortiline dans les cellules épithéliales mammaires normales que dans les cancéreuses.

### Diminution de l'expression de la Sortiline par ARN interférence (Sortiline knock down)

Les cellules MDA-MB-231 maintenues dans le milieu de culture EMEM ont été transfectées soit avec du milieu de culture seul (Mock), soit avec un ARN interférant dirigé contre la protéine GFP (siGFP) ou avec un ARN interférant dirigé contre la Sortiline (siSORT).

Les résultats présentés sur là figure 7 montrent que dans les conditions de culture choisies, le nombre de cellules MDA-MB-231 en culture n'augmente pas au cours du temps en présence du siSORT. Dans les groupes contrôles Mock ou siGFP, le nombre de cellules double entre 24 et 48h de culture. La prolifération de la lignée mammaire cancéreuse MDA-MB-231 est donc ralentie par transfection d'un ARN interférant dirigée contre la Sortiline. Nous avons vérifié par Western blot que la transfection du siSORT diminuait bien le niveau d'expression de la protéine Sortiline. Ainsi, cette expérience nous a permis de montrer qu'il était possible de contrôler la prolifération anormale des cellules du cancer de sein en ciblant la Sortiline, le récepteur du ProNGF, par un ARN interférant.

### Migration

Les effets du ProNGF sur la migration des cellules épithéliales mammaires ont été appréhendés par des tests en chambre de Boyden (transwell®). Chaque condition est réalisée en duplicate et un minimum de 40 champs est compté. Les MCF-7 sont ensemencées dans du sevrage à la face supérieure du transwell® alors que la face inférieure baignent dans du milieu de sevrage seul ou avec 200 ng/mL de ProNGF, ou avec 20 µM de galardine avec ou sans 200 ng/mL de ProNGF, ou avec 200 ng/mL de NGF. Les cellules ayant traversées le transwell® sont ensuite comptées. *p<0,05, comparaison avec la condition sevrage seul.

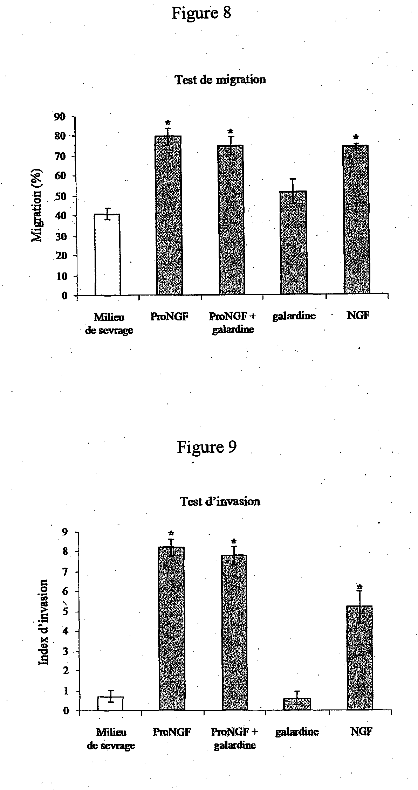

Les résultats sont indiqués sur la figure 8 qui représente un graphe donnant le pourcentage de migration cellulaire à partir de cellules MCF-7 baignant dans les différents milieux. Ces résultats mettent en évidence qu'un traitement des cellules cancéreuses MCF-7 par du ProNGF permet d'augmenter leur capacité à migrer et ceci de façon semblable au NGF à doses égales. L'addition au milieu de culture de galardine, qui est capable d'inhiber la synthèse de NGF à partir de ProNGF, tend à démontrer que la promotion de l'activité migratoire est due au ProNGF et non au NGF qu'il est capable de générer.

### Invasion

Le pouvoir invasif des MCF-7 stimulées par le ProNGF a été testé en gel de collagène de type I. Chaque condition est réalisée en triplicate et un minimum de 45 champs est compté par condition. Les MCF-7 sont ensemencées sur un gel de collagène de type I dans du milieu de sevrage seul ou avec 200 ng/mL de ProNGF, ou avec 20 µM de galardine avec ou sans 200 ng/mL de ProNGF, ou avec 200 ng/mL de NGF. *p<0,05, comparaison avec la condition sevrage seul.

Les résultats sont indiqués sur la figure 9 qui représente un graphe donnant l'index d'invasion à partir de cellules MCF-7 baignant dans les différents milieux. Ces tests montrent que le ProNGF stimule l'invasion des MCF-7.

### Diminution de l'expression du ProNGF par ARN interférence

Les cellules MCF-7 ont été transfectées par 3 µg d'ARN interférant (siRNA) dirigé soit contre la GFP (siGFP), soit contre le ProNGF (siProNGF). Au bout de 24 à 48h de culture, les cellules ont été lysées et un Western-blot ProNGF a été réalisé. La quantité relative de ProNGF détectée en blot a été évaluée avec le logiciel QuantityOne (Bio-Rad) rapportée à l'équicharge actine et présentée sous forme d'histogramme où 100% est attribué à la condition contrôle siGFP (Figure 10). Sur les photographies des blots nous avons déjà pu observer que la transfection du siProNGF diminue l'expression de là protéine ProNGF dans les cellules MCF-7. L'analyse densitométrique a permis d'évaluer cette diminution à 59% au bout de 24h de transfection.

Cette expérience nous a permis de montrer qu'il était possible de diminuer le niveau d'expression du ProNGF dans des cellules du cancer de sein en utilisant la stratégie de l'ARN interférence.

## Revendications

1. Procédé de diagnostic *in vitro* du cancer, **caractérisé en ce qu'**il consiste à déterminer la présence de ProNGF dans un échantillon biologique issu d'un patient suspecté d'être atteint du cancer.

2. Procédé de diagnostic *in vitro* du cancer selon la revendication 1, **caractérisé en ce que** le cancer est un cancer du sein, de la thyroïde ou du poumon.

3. Procédé de diagnostic *in vitro* du cancer selon la revendication 2, **caractérisé en ce que** le cancer est un cancer du sein.

4. Procédé de diagnostic *in vitro* du cancer selon la revendication 2, **caractérisé en ce que** le cancer est un cancer du poumon.

5. Procédé de diagnostic *in vitro* du cancer selon la revendication 2, **caractérisé en ce que** le cancer est un cancer de la thyroïde.

6. Procédé selon l'une des revendications 1 à 5, **caractérisé en ce que** la présence de ProNGF est mise en évidence par détection directe du ProNGF dans ledit échantillon biologique.

7. Procédé selon la revendication 6, **caractérisé en ce que** la détection du ProNGF est mise en oeuvre par un test immunologique ou par spectrométrie de masse.

8. Procédé selon l'une des revendications 6 ou 7, **caractérisé en ce que** ledit échantillon biologique est constitué de fluide biologique ou d'un tissu provenant de la biopsie de la tumeur ou de métastases.

9. Procédé selon la revendication 8, **caractérisé en ce que** l'échantillon biologique est constitué d'un tissu provenant de la biopsie de la tumeur ou des métastases du patient.

10. Procédé selon la revendication 8, **caractérisé en ce que** l'échantillon biologique est constitué de fluide biologique, de préférence préalablement traité pour isoler les cellules tumorales circulantes contenues dans ledit fluide.

11. Procédé selon la revendication 10, **caractérisé en ce que** les cellules tumorales circulantes sont ensuite cultivées en conditions telles qu'elles sécrètent du ProNGF.

12. Procédé selon l'une des revendications 10 ou 11, **caractérisé en ce que** les cellules tumorales circulantes sont également cultivées dans des conditions permettant le blocage du ProNGF à l'intérieur desdites cellules.

13. Procédé selon l'une des revendications 1 à 5, **caractérisé en ce que** la détection de ProNGF est mise en évidence par culture, en présence dudit échantillon biologique, de cellules sensibles au ProNGF.

14. Procédé selon la revendication 13, **caractérisé en ce que** ledit échantillon biologique est constitué d'un échantillon de fluide biologique, de préférence préalablement traité pour isoler les cellules tumorales circulantes contenues dans ledit fluide.

15. Procédé selon la revendication 14, **caractérisé en ce que** les cellules tumorales circulantes sont ensuite cultivées en conditions telles qu'elles sécrètent du ProNGF.

16. Utilisation du procédé selon l'une quelconque des revendications 1 à 15 dans le diagnostic précoce, le dépistage, le suivi thérapeutique, le pronostic et le diagnostic des rechutes dans le cadre du cancer, ledit cancer étant de préférence le cancer du sein, de la thyroïde ou du poumon.

17. Utilisation d'un inhibiteur du ProNGF pour la préparation d'un médicament destiné au traitement du cancer du sein, de la thyroïde, de la prostate, ou du poumon; **caractérisée en ce que** l'inhibiteur est un anticorps spécifique du ProNGF, les fractions d'anticorps spécifique du ProNGF, un analogue sous forme soluble d'un récepteur au ProNGF, un SiRNA d'un récepteur du ProNGF, un oligonucléotide anti-sens dudit récepteur, un peptide dérivé du ProNGF ayant conservé les propriétés de liaison au récepteur du ProNGF ou un anticorps spécifique dirigé contre le récepteur du ProNGF.

18. Utilisation selon la revendication 17, **caractérisée en ce que** ledit inhibiteur du ProNGF a été préalablement mis en conditions telles qu'il pénètre spécifiquement dans les cellules d'intérêt.

19. Composition pharmaceutique, **caractérisée en ce qu'**elle comprend à titre de principe actif au moins un inhibiteur du ProNGF, pour son utilisation dans le traitement du cancer du sein, de la thyroïde, de la prostate, ou du poumon, **caractérisée en ce que** l'inhibiteur est un anticorps spécifique anti-ProNGF, les fractions d'anticorps spécifique anti-ProNGF, un analogue sous forme soluble d'un récepteur au ProNGF, un SiRNA d'un récepteur du ProNGF ou un oligonucléotide anti-sens dudit récepteur, un peptide dérivé du ProNGF ayant conservé les propriétés de liaison au dit récepteur ou un anticorps dirigé contre le récepteur du ProNGF.

## Patentansprüche

1. *In-vitro*-Verfahren zur Diagnose von Krebs, **dadurch gekennzeichnet, dass** es aus der Bestimmung des Vorliegens von ProNGF in einer biologischen Probe von einem Patienten, bei dem Verdacht auf Krebs besteht, besteht.

2. *In-vitro*-Verfahren zur Diagnose von Krebs nach Anspruch 1, **dadurch gekennzeichnet, dass** es sich bei dem Krebs um einen Brustkrebs, einen Schilddrüsenkrebs oder einen Lungenkrebs handelt.

3. *In-vitro*-Verfahren zur Diagnose von Krebs nach Anspruch 2, **dadurch gekennzeichnet, dass** es sich bei dem Krebs um einen Brustkrebs handelt.

4. *In-vitro*-Verfahren zur Diagnose von Krebs nach Anspruch 2, **dadurch gekennzeichnet, dass** es sich bei dem Krebs um einen Lungenkrebs handelt.

5. *In-vitro*-Verfahren zur Diagnose von Krebs nach Anspruch 2, **dadurch gekennzeichnet, dass** es sich bei dem Krebs um einen Schilddrüsenkrebs handelt.

6. Verfahren nach einem der Ansprüche 1 bis 5, **dadurch gekennzeichnet, dass** das Vorliegen von ProNGF dadurch nachgewiesen wird, dass man ProNGF in der biologischen Probe direkt nachweist.

7. Verfahren nach Anspruch 6, **dadurch gekennzeichnet, dass** ProNGF durch einen immunologischen Test oder mittels Massenspektrometrie nachgewiesen wird.

8. Verfahren nach einem der Ansprüche 6 oder 7, **dadurch gekennzeichnet, dass** die biologische Probe aus biologischer Flüssigkeit oder aus Gewebe, das aus der Biopsie des Tumors oder von Metastasen stammt, besteht.

9. Verfahren nach Anspruch 8, **dadurch gekennzeichnet, dass** die biologische Probe aus Gewebe, das aus der Biopsie des Tumors oder von Metastasen des Patienten stammt, besteht.

10. Verfahren nach Anspruch 8, **dadurch gekennzeichnet, dass** die biologische Probe aus biologischer Flüssigkeit, die vorzugsweise zuvor behandelt worden ist, um die in dieser Flüssigkeit zirkulierenden Tumorzellen zu isolieren, besteht.

11. Verfahren nach Anspruch 10, **dadurch gekennzeichnet, dass** die zirkulierenden Tumorzellen anschließend unter Bedingungen, unter denen sie ProNGF sezernieren, kultiviert werden.

12. Verfahren nach einem der Ansprüche 10 oder 11, **dadurch gekennzeichnet, dass** die zirkulierenden Tumorzellen auch unter Bedingungen kultiviert werden, die eine Blockierung des ProNGF im Inneren dieser Zellen gestatten.

13. Verfahren nach einem der Ansprüche 1 bis 5, **dadurch gekennzeichnet, dass** der ProNGF-Nachweis durch Kultivieren von ProNGF-sensitiven Zellen in Gegenwart der biologischen Probe erfolgt.

14. Verfahren nach Anspruch 13, **dadurch gekennzeichnet, dass** die biologische Probe aus einer Probe biologischer Flüssigkeit, die vorzugsweise zuvor behandelt worden ist, um die in dieser Flüssigkeit zirkulierenden Tumorzellen zu isolieren, besteht.

15. Verfahren nach Anspruch 14, **dadurch gekennzeichnet, dass** die zirkulierenden Tumorzellen anschließend unter Bedingungen, unter denen sie ProNGF sezernieren, kultiviert werden.

16. Verwendung des Verfahrens nach einem der Ansprüche 1 bis 15 in der Frühdiagnose, der Erkennung, der Nachsorge, der Prognose und der Diagnose von Rückfällen in Zusammenhang mit Krebs, wobei es sich bei dem Krebs vorzugsweise um Brustkrebs, Schilddrüsenkrebs oder Lungenkrebs handelt.

17. Verwendung eines ProNGF-Hemmers für die Herstellung eines Arzneimittels zur Behandlung von Brustkrebs, Schilddrüsenkrebs, Prostatakrebs oder Lungenkrebs, **dadurch gekennzeichnet, dass** es sich bei dem Hemmer um einen Antikörper mit Spezifität für ProNGF, Antikörperfraktionen mit Spezifität für ProNGF, ein ProNGF-Rezeptoranalog in löslicher Form, eine SiRNA eines ProNGF-Rezeptors, ein antisense-Oligonukleotid dieses Rezeptors, ein von ProNGF abgeleitetes Peptid, bei dem die ProNGF-Rezeptorbindungseigenschaften konserviert sind, oder einen spezifischen Antikörper gegen den ProNGF-Rezeptor handelt.

18. Verwendung nach Anspruch 17, **dadurch gekennzeichnet, dass** der ProNGF-Hemmer zuvor solchen Bedingungen ausgesetzt wurde, dass er spezifisch in die interessierenden Zellen eindringt.

19. Pharmazeutische Zusammensetzung, **dadurch gekennzeichnet, dass** sie als Wirkprinzip mindestens einen ProNGF-Hemmer umfasst, für seine Verwendung in der Behandlung von Brustkrebs, Schilddrüsenkrebs, Prostatakrebs oder Lungenkrebs, **dadurch gekennzeichnet, dass** es sich bei dem Hemmer um einen Antikörper mit Spezifität für ProNGF, Antikörperfraktionen mit Spezifität für ProNGF, ein ProNGF-Rezeptoranalog in löslicher Form, eine SiRNA eines ProNGF-Rezeptors, ein antisense-Oligonukleotid dieses Rezeptors, ein von ProNGF abgeleitetes Peptid, bei dem die Bindungseigenschaften an diesen Rezeptor konserviert sind, oder einen Antikörper gegen den ProNGF-Rezeptor handelt.

## Claims

1. A method for *in vitro* diagnosis of cancer, **characterized in that** it consists in determining the presence of ProNGF in a biological sample derived from a patient suspected of suffering from cancer.

2. The method for *in vitro* diagnosis of cancer as claimed in claim 1, **characterized in that** the cancer is breast, thyroid or lung cancer.

3. The method for *in vitro* diagnosis of cancer as claimed in claim 2, **characterized in that** the cancer is breast cancer.

4. The method for *in vitro* diagnosis of cancer as claimed in claim 2, **characterized in that** the cancer is lung cancer.

5. The method for *in vitro* diagnosis of cancer as claimed in claim 2, **characterized in that** the cancer is thyroid cancer.

6. The method as claimed in one of claims 1 to 5, **characterized in that** the presence of ProNGF is demonstrated by direct detection of ProNGF in said biological sample.

7. The method as claimed in claim 6, **characterized in that** the detection of ProNGF is carried out by means of an immunoassay or by mass spectrometry.

8. The method as claimed in either of claims 6 and 7, **characterized in that** said biological sample consists of biological fluid or of a tissue originating from the biopsy of the tumor or metastases.

9. The method as claimed in claim 8, **characterized in that** the biological sample consists of a tissue originating from the biopsy of the tumor or the metastases of the patient.

10. The method as claimed in claim 8, **characterized in that** the biological sample consists of biological fluid, preferably pretreated in order to isolate the circulating tumor cells contained in said fluid.

11. The method as claimed in claim 10, **characterized in that** the circulating tumor cells are subsequently cultured under conditions such that they secrete ProNGF.

12. The method as claimed in either of claims 10 and 11, **characterized in that** the circulating tumor cells are also cultured under conditions which make it possible to block the ProNGF inside said cells.

13. The method as claimed in one of claims 1 to 5, **characterized in that** the detection of ProNGF is carried out by culturing ProNGF-sensitive cells in the presence of said biological sample.

14. The method as claimed in claim 13, **characterized in that** said biological sample consists of a biological fluid sample, preferably pretreated in order to isolate the circulating tumor cells contained in said fluid.

15. The method as claimed in claim 14, **characterized in that** the circulating tumor cells are subsequently cultured under conditions such that they secrete ProNGF.

16. The use of the method as claimed in any one of claims 1 to 15 in early diagnosis, screening, therapeutic follow-up, prognosis and diagnosing relapses in cancer, said cancer preferably being breast, thyroid or lung cancer.

17. The use of a ProNGF inhibitor for preparing a drug for use in the treatment of breast, thyroid, prostate or lung cancer, **characterized in that** the inhibitor is specific ProNGF antibody, specific ProNGF antibody fractions, an analog, in soluble form, of ProNGF receptor, an siRNA of a ProNGF receptor, an antisense oligonucleotide of said receptor, a ProNGF-derived peptide which has conserved the properties of binding to the ProNGF receptor or a specific antibody directed against the ProNGF receptor.

18. The use as claimed in claim 17, **characterized in that** said ProNGF inhibitor has been previously placed under conditions such that it specifically penetrates into the cells of interest.

19. Pharmaceutical composition, **characterized in that** it comprises as active ingredient, at least one ProNGF inhibitor for use in the treatment of breast, thyroid, prostate or lung cancer, **characterized in that** the inhibitor is specific ProNGF antibody, specific ProNGF antibody fractions, an analog, in soluble form, of ProNGF receptor, an siRNA of a ProNGF receptor, an antisense oligonucleotide of said receptor, a ProNGF-derived peptide which has conserved the properties of binding to the ProNGF receptor or a specific antibody directed against the ProNGF receptor.


### SEQUENCE LISTING

<110> bioMérieux Université des Sciences et Technologies de Lille
<120> Procédé de dosage du ProNGF pour le diagnostic *in vitro* du cancer en particulier du cancer du sein, de la thyroïde ou du poumon et utilisation du ProNGF en thérapie
<130> ProNGF
<150> FR0600851
   <151> 2006-01-31
<160> 6
<170> PatentIn version 3.3
<210> 1
   <211> 21
   <212> RNA
   <213> Artificial
<220>
   <223> siGFP : in the sequence n represents t (thymine)
<220>
   <221> misc_feature
   <222> (20)..(21)
   <223> n is a, c, g, or u
<400> 1
   gcugacccug aaguucaucn n 21
<210> 2
   <211> 21
   <212> RNA
   <213> Artificial
<220>
   <223> siGFP : in the sequence n represents t (thymine)
<220>
   <221> misc_feature
   <222> (20)..(21)
   <223> n is a, c, g, or u
<400> 2
   gaugaacuuc agggucagcn n 21
<210> 3
   <211> 21
   <212> RNA
   <213> Artificial
<220>
   <223> siSORT : in the sequence n represents t (thymine)
<220>
   <221> misc_feature
   <222> (20)..(21)
   <223> n is a, c, g, or u
<400> 3
   ggugguguua acagcagagn n 21
<210> 4
   <211> 21
   <212> RNA
   <213> Artificial
<220>
   <223> siSORT : in the sequence n represents t (thymine)
<220>
   <221> misc_feature
   <222> (20)..(21)
   <223> n is a, c, g, or u
<400> 4
   cucugcuguu aacaccaccn n 21
<210> 5
   <211> 21
   <212> RNA
   <213> Artificial
<220>
   <223> siProNGF : in the sequence n represents t (thymine)
<220>
   <221> misc_feature
   <222> (20)..(21)
   <223> n is a, c, g, or u
<400> 5
   caguguauuc aaacaguaun n 21
<210> 6
   <211> 21
   <212> RNA
   <213> Artificial
<220>
   <223> siProNGF : in the sequence n represents t -thymine)
<220>
   <221> misc_feature
   <222> (20)..(21)
   <223> n is a, c, g, or u
<400> 6
   guacuguuug aauacacugn n 21